# EUROPEAN PATENT APPLICATION

(11) **EP 3 103 798 A1**
(43) Date of publication of application: **14.12.2016**
(21) Application number: 15171097.7
(22) Date of filing: 09.06.2015
(51) Int. Cl.: C07D 239/34, A01N 43/54

(54) **HERBICIDAL FLUOROMETHANESULFONAMIDES**

(71) Applicant: BASF SE, 67056 Ludwigshafen am Rhein (DE)
(72) Inventor: Seitz, Thomas, 68519 Viernheim (DE); Vogt, Florian, 68167 Mannheim (DE); Kraus, Helmut, 67160 Wissembourg (FR); Witschel, Matthias, 67098 Bad Dürkheim (DE); Seiser, Tobias, 68163 Mannheim (DE); Michrowska-Pianowska, Anna Aleksandra, 68167 Mannheim (DE); Major, Julia, 67251 Freinsheim (DE); Mietzner, Thomas, 76855 Annweiler (DE); Sisay, Mihiret Tekeste, 68163 Mannheim (DE); Tresch, Stefan, 67281 Kirchheim (DE); Parra Rapado, Liliana, 77654 Offenburg (DE); Krämer, Gerd, 67304 Kerzenheim (DE); Newton, Trevor William, 67435 Neustadt (DE); Evans, Richard Roger, 27613 Raleigh , (US)
(74) Representative: BASF IP Association

(57) **Abstract**

The present invention relates to novel fluoromethanesulfonamides of the formula (I) or an N-oxide or a salt thereof or an acid addition salt thereof; to an agrochemical composition, which comprises an auxiliary and at least one compound of formula (I) or an N-oxide or a salt thereof or an acid addition salt thereof; to the use of compounds of formula (I), their N-oxides or their salts or their acid addition salts for controlling unwanted vegetation; and to a method for controlling unwanted vegetation, which comprises allowing a herbicidally effective amount of at least one compound of formula (I) or a salt thereof or an acid adition salt thereof, or an agrochemical composition, to act on plants, their seeds and/or their habitat

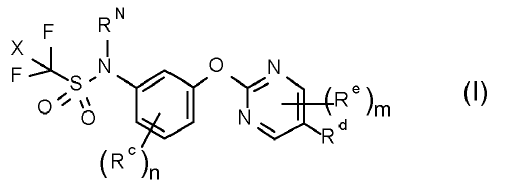

## Description

The present invention relates to novel fluoromethanesulfonamides of the formula (I) or an N-oxide or a salt thereof or an acid addition salt thereof; to an agrochemical composition, which comprises an auxiliary and at least one compound of formula (I) or an N-oxide or a salt thereof or an acid addition salt thereof; to the use of compounds of formula (I), their N-oxides or their salts or their acid addition salts for controlling unwanted vegetation; and to a method for controlling unwanted vegetation, which comprises allowing a herbicidally effective amount of at least one compound of formula (I) or a salt thereof or an acid adition salt thereof, or an agrochemical composition, to act on plants, their seeds and/or their habitat

US 435077 relates to methanesulfonamide compounds with a meta-relationship on the central phenyl ring between the sulfonamide group and the hetaryloxy group and to their use to control unwanted vegetation. US 435076, JP 8193011 and JP 8291146 also relate to herbicidal methanesulfonamide compounds bearing inter alia a 2-pyrimidyl radical, however, featuring a para-relationship on the central phenyl ring between the sulfonamide group and the hetaryloxy group.

The herbicidal properties of these known compounds with regard to the harmful plants are not always entirely satisfactory. It is therefore an object of the present invention to provide fluoromethanesulfonamides of the formula (I) having improved herbicidal action. To be provided are in particular fluoromethanesulfonamides of the formula (I) which have high herbicidal activity, in particular even at low application rates, and which are sufficiently compatible with crop plants for commercial utilization. These and further objects are achieved by fluoromethanesulfonamides of the formula (I) as defined below and by their agriculturally suitable salts. The compounds according to the invention differ from those described in the closest prior art US 435077 in that they feature a halogenated pyrimidine ring instead of a pyridazine ring.

Accordingly, the present invention relates compounds of the formula (I) or their N-oxides or their salts or acid addition salts, 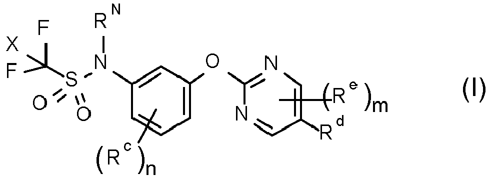 wherein:
- X: is hydrogen, halogen or C₁-C₆-haloalkyl;
- R^{N}: is hydrogen, C₁-C₆-alkyl, C₁-C₆-alkoxy-C₁-C₆-alkyl, C₂-C₆-alkenyl, C₂-C₆-alkynyl, C₃-C₆-cycloalkyl, phenyl-C₁-C₄-alkyl, heteroaryl-C₁-C₄-alkyl, (C=O)-(C₁-C₆-alkyl), (C=O)- C₂-C₆-alkynyl, (C=O)-(C₃-C₆-cycloalkyl), (C=O)-(C₁-C₆-alkoxy), (C=O)-(C₂-C₆-alkynyloxy), (C=O)-(C₁-C₆-alkylthio), (C=O)-(C₁-C₆-alkylamino), (C=O)-(C₁-C₆-dialkylamino), (C=O)-phenyl, (C=O)-heteroaryl, (C=O)-(phenyl-C₁-C₄-alkyl), (C=O)-(heteroaryl-C₁-C₄-alkyl), C₁-C₆-alkylsulfonyl, C₃-C₆-cycloalkylsulfonyl, C₁-C₆-alkoxysulfonyl, C₁-C₆-alkylaminosulfonyl, C₁-C₆-dialkylaminosulfonyl, phenylsulfonyl, heteroarylsulfonyl, C₁-C₆-alkyl-(C=O)-O-C₁-C₆-alkyl, C₃-C₆-cycloalkyl-(C=O)-O-C₁-C₆-alkyl, C₁-C₆-haloalkyl-(C=O)-O-C₁-C₆-alkyl, C₂-C₆-alkenyl-(C=O)-O-C₁-C₆-alkyl, C₂-C₆-alkynyl-(C=O)-O-C₁-C₆-alkyl, C₁-C₆-alkoxy-(C=O)-O-C₁-C₆-alkyl, C₁-C₆-haloalkoxy-(C=O)-O-C₁-C₆-alkyl, C₂-C₆-alkenyloxy-(C=O)-O-C₁-C₆-alkyl or C₂-C₆-alkynyloxy-(C=O)-O-C₁-C₆-alkyl; and wherein the aliphatic and cyclic moieties of R^{N} are unsubstituted or substituted by 1, 2, 3 or up to the maximum number of identical or different groups R^{N1}; wherein
R^{N1} is halogen, hydroxy, cyano, nitro, C₁-C₄-alkyl, C₁-C₄-alkoxy, C₁-C₄-alkylthio, C₁-C₄-alkylsulfinyl, C₁-C₄-alkylsulfonyl, C₁-C₄-alkylamino, C₁-C₄-dialkylamino, C₃-C₆-cycloalkyl, C₁-C₄-haloalkyl or C₁-C₄-haloalkoxy;
- R^{c}: is independently selected from the group consisting of halogen, cyano, nitro, hydroxy, SH, NH₂, C₁-C₆-alkyl, C₁-C₆-alkoxy, C₁-C₆-haloalkyl, C₁-C₆-haloalkoxy, C₁-C₄-alkoxy-C₁-C₄-alkyl, C₁-C₆-alkylthio, C₁-C₆-haloalkylthio, C₁-C₆-alkylsulfinyl, C₁-C₆-alkylsulfonyl, C₁-C₆-haloalkylsulfonyl, C₁-C₆-alkylamino, C₁-C₆-dialkylamino, C₁-C₆-alkyl-(C=O)-amino, C₁-C₆-alkylsulfonylamino, C₁-C₆-alkyl-(C=O)-(C₁-C₆-alkylamino), (C₁-C₆-alkylsulfonyl)-(C₁-C₆-alkylamino), C₂-C₆-alkenyl, C₂-C₆-alkynyl, C₂-C₆-alkenyloxy, C₂-C₆-alkynyloxy, C₃-C₈-cycloalkyl, C₃-C₈-cycloalkoxy, (C=O)-OH, (C=O)-NH₂, (C=O)-(C₁-C₆-alkyl), (C=O)-(C₁-C₆-alkenyl), (C=O)-(C₁-C₆-alkynyl), (C=O)-(C₁-C₆-alkoxy), (C=O)-(C₁-C₆-alkenyloxy), (C=O)-(C₁-C₆-alkynyloxy), (C=O)-(C₁-C₆-alkylthio), (C=O)-(C₁-C₆-alkylamino), (C=O)-(C₁-C₆-dialkylamino), (C=N-O-(C₁-C₆-alkyl))-(C₁-C₆-alkyl), (CH=N-O-(C₁-C₆-alkyl)) and (C=N-O-(C₁-C₆-alkyl))-(C₁-C₆-alkoxy); and wherein the aliphatic and alicyclic moieties are unsubstituted or substituted by 1, 2, 3 or up to the maximum number of identical or different groups R^{c1}; wherein
R^{c1} is halogen, hydroxy, cyano, nitro, C₁-C₄-alkyl, C₁-C₄-alkoxy, C₁-C₄-alkylthio, C₁-C₄-alkylsulfinyl, C₁-C₄-alkylsulfonyl, C₁-C₄-alkylamino, C₁-C₄-dialkylamino, C₃-C₆-cycloalkyl, C₁-C₄-haloalkyl or C₁-C₄-haloalkoxy;
- n: is 0, 1, 2 or 3;
- Re: is independently selected from the group consisting of halogen, cyano, nitro, hydroxy, SH, NH₂, C₁-C₆-alkyl, C₁-C₆-alkoxy, C₁-C₆-haloalkyl, C₁-C₆-haloalkoxy, C₁-C₄-alkoxy-C₁-C₄-alkyl, C₁-C₆-alkylthio, C₁-C₆-haloalkylthio, C₁-C₄-alkylthio-C₁-C₄-alkyl, C₁-C₆-alkylsulfinyl, C₁-C₆-alkylsulfonyl, C₁-C₆-alkylamino, C₁-C₆-dialkylamino, C₁-C₆-alkyl-(C=O)-amino, C₂-C₆-alkenyl, C₂-C₆-alkynyl, C₃-C₈-cycloalkyl, C₃-C₈-cycloalkoxy, (C=O)-OH, (C=O)-NH₂, (C=O)-(C₁-C₆-alkyl), (C=O)-(C₁-C₆-alkoxy), (C=O)-(C₁-C₆-alkylthio), (C=O)-(C₁-C₆-alkylamino), (C=O)-(C₁-C₆-dialkylamino);
- m: is 0, 1 or 2;
- R^{d}: is independently selected from the group consisting of fluorine, chlorine or bromine.

The present invention also provides agrochemical compositions comprising at least one compound of the formula (I) and auxiliaries customary for formulating crop protection agents. The present invention also provides the use of compounds of the formula (I) as herbicides, i.e. for controlling harmful plants.

The present invention furthermore provides a method for controlling unwanted vegetation where a herbicidal effective amount of at least one compound of the formula (I) is allowed to act on plants, their seeds and/or their habitat. Application can be done before, during and/or after, preferably during and/or after, the emergence of the undesirable plants.

If the herbicidal compounds of the formula (I) as described herein have ionizable functional groups, they can also be employed in the form of their agriculturally acceptable salts. These can be obtained by way of abstraction of a proton from compounds of the formula (I) with a base. Acid addition salts can be formed by protonating a compound of the formua (I) with an acid. Suitable in the sense of the term "agriculturally acceptable" are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl phenyl or benzyl, preferably ammonium, methylammonium, isopropylammonium, dimethylammonium, diisopropylammonium, trimethylammonium, heptylammonium, dodecylammonium, tetradecylammonium, tetramethylammonium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri(C₁-C₄-alkyl)sulfonium, such as trimethylsulfonium, and sulfoxonium ions, preferably tri(C₁-C₄-alkyl)sulfoxonium, and finally the salts of polybasic amines such as N,N-bis-(3-aminopropyl)methylamine and diethylenetriamine.

Anions of useful acid addition salts are primarily chloride, bromide, fluoride, iodide, hydrogensulfate, methylsulfate, sulfate, dihydrogenphosphate, hydrogenphosphate, nitrate, bicarbonate, carbonate, hexafluorosilicate, hexafluorophosphate, benzoate and also the anions of C₁-C₄-alkanoic acids, preferably formate, acetate, propionate and butyrate. They can be formed by reacting a compound I with an acid of the corresponding anion, preferably of hydrochloric acid, hydrobromic acid, sulfuric acid, phosphoric acid or nitric acid.

Compounds of formula (I) as described herein having a carboxyl group can be employed in the form of the acid, in the form of an agriculturally suitable salt as mentioned above or else in the form of an agriculturally acceptable derivative, for example as amides, such as mono- and di-C₁-C₆-alkylamides or arylamides, as esters, for example as allyl esters, propargyl esters, C₁-C₁₀-alkyl esters, alkoxyalkyl esters, tefuryl ((tetrahydrofuran-2-yl)methyl) esters and also as thioesters, for example as C₁-C₁₀-alkylthio esters. Preferred mono- and di-C₁-C₆-alkylamides are the methyl and the dimethylamides. Preferred arylamides are, for example, the anilides and the 2-chloroanilides. Preferred alkyl esters are, for example, the methyl, ethyl, propyl, isopropyl, butyl, isobutyl, pentyl, mexyl (1-methylhexyl), meptyl (1-methylheptyl), heptyl, octyl or isooctyl (2-ethylhexyl) esters. Preferred C₁-C₄-alkoxy-C₁-C₄-alkyl esters are the straight-chain or branched C₁-C₄-alkoxy ethyl esters, for example the 2-methoxyethyl, 2-ethoxyethyl, 2-butoxyethyl (butotyl), 2-butoxypropyl or 3-butoxypropyl ester. An example of a straight-chain or branched C₁-C₁₀-alkylthio ester is the ethylthio ester.

Compounds of the formula (I) can exist as one or more stereoisomers. The various stereoisomers include enantiomers, diastereomers, atropisomers arising from restricted rotation about a single bond of asymmetric groups and geometric isomers. They also form part of the subject matter of the present invention. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. The compounds of the invention may be present as a mixture of stereoisomers, e.g. a racemate, individual stereoisomers, or as an optically active form.

Compounds of the formula (I) can be present in different crystal modifications whose biological activity may differ. They also form part of the subject matter of the present invention.

As used herein, the terms "controlling" and "combating" are synonyms.

As used herein, the terms "undesirable vegetation" and "harmful plants" are synonyms.

In respect of the variables, the embodiments of the intermediates obtained during preparation of compounds I correspond to the embodiments of the compounds of the formula (I). The term "compounds I" refers to compounds of the formula (I).

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, for example methyl, ethyl, propyl, 1-methylethyl, butyl, 1-methylpropyl, 2-methylpropyl, and 1,1-dimethylethyl.

The term "C₁-C₆-haloalkyl" refers to a straight-chained or branched alkyl group having 1 to 6 carbon atoms (as defined above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "C₁-C₆-alkoxy" refers to a straight-chain or branched alkyl group having 1 to 6 carbon atoms (as defined above) which is bonded via an oxygen, at any position in the alkyl group, for example methoxy (or OCH₃), ethoxy, n-propoxy, 1-methylethoxy, butoxy, 1-methylpropoxy, 2-methylpropoxy or 1,1-dimethylethoxy.

The term "C₁-C₆-haloalkoxy" refers to a C₁-C₆-alkoxy group as defined above, wherein some or all of the hydrogen atoms may be replaced by halogen atoms as mentioned above, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromo¬ethoxy, 4-fluorobutoxy, 4-chlorobutoxy, 4-bromobutoxy or nonafluorobutoxy.

The terms "phenyl-C₁-C₄-alkyl or heteroaryl-C₁-C₄-alkyl" refer to an alkyl radical having 1 to 4 carbon atoms (as defined above), wherein one hydrogen atom of the alkyl radical is replaced by a phenyl or hetereoaryl radical respectively.

The term "C₁-C₆-alkoxy-C₁-C₆-alkyl" refers to an alkyl radical having 1 to 6 carbon atoms (as defined above), wherein one hydrogen atom of the alkyl radical is replaced by a C₁-C₆-alkoxy group (as defined above).

The terms "C₁-C₆-alkylamino" or "C₁-C₆-dialkylamino" refer to one or two (respectively) C₁-C₆-alkyl radicals, which are bound through an amino group, or otherwise denoted as C₁-C₆-alkyl-NH- or -N(C₁-C₆-alkyl)₂.

The term "C₁-C₆-alkylthio" as used herein refers to straight-chain or branched alkyl groups having 1 to 6 carbon atoms (as defined above) bonded via a sulfur atom. Accordingly, the term "C₁-C₆-haloalkylthio" as used herein refers to straight-chain or branched haloalkyl group having 1 to 6 carbon atoms (as defined above) bonded through a sulfur atom, at any position in the haloalkyl group.

The term "C₁-C₆-alkylsulfinyl" refers to straight-chain or branched alkyl groups having 1 to 6 carbon atoms (as defined above) bonded through a -S(=O)- moiety, at any position in the alkyl group, for example methylsulfinyl and ethylsulfinyl, and the like. Accordingly, the term "C₁-C₆-haloalkylsulfinyl" refers to straight-chain or branched haloalkyl group having 1 to 6 carbon atoms (as defined above), bonded through a -S(=O)- moiety, at any position in the haloalkyl group.

The term "C₁-C₆-alkylsulfonyl" refers to straight-chain or branched alkyl groups having 1 to 6 carbon atoms (as defined above), bonded through a -S(=O)₂- moiety, at any position in the alkyl group, for example methylsulfonyl. Accordingly, the term "C₁-C₆-haloalkylsulfonyl" refers to straight-chain or branched haloalkyl group having 1 to 6 carbon atoms (as defined above), bonded through a -S(=O)₂- moiety, at any position in the haloalkyl group.

The term "C₁-C₆-alkoxysulfonyl" refers to straight-chain or branched alkoxy group having 1 to 6 carbon atoms (as defined above), bonded through a -S(=O)₂- moiety, or otherwise denoted as C₁-C₆-alkyl-O-S(=O)₂-.

The terms "C₁-C₆-alkylaminosulfonyl" or "C₁-C₆-dialkylaminosulfonyl" "refer to amino groups that are substituted with one or two (respectively) straight-chain or branched C₁-C₆-alkyl groups, at any position in the alkyl group, and which are bonded through a -S(=O)₂- moiety.

The terms "C₁-C₆-alkylsulfonylamino" or "(C₁-C₆-alkylsulfonyl)-(C₁-C₆-alkylamino)" refer to a C₁-C₆-alkylsulfonyl group bonded through an amino group, which is unsubstitued or substituted by a C₁-C₆-alkyl group respectively.

The terms "phenylsulfonyl" or "heteroarylsulfonyl" refer to an aromatic ring (as defined herein), bonded through a -S(=O)₂- moiety.

The term "(CH=N-O-(C₁-C₆-alkyl))" refers to a radical which is attached through the carbon atom of the (C=N-O-) group as indicated by the number valence of the carbon atom, and which is further substituted by a hydrogen atom.

The term "(C=N-O-(C₁-C₆-alkyl))-(C₁-C₆-alkyl)" )" refers to a radical which is attached through the carbon atom of the (C=N-O-) group as indicated by the number valence of the carbon atom, and which is further substituted by a C₁-C₆-alkyl group.

The term "(C=N-O-(C₁-C₆-alkyl))-(C₁-C₆-alkoxy)" refers to a radical which is attached through the carbon atom of the (C=N-O-) group as indicated by the number valence of the carbon atom, and which is further substituted by a C₁-C₆-alkoxy group.

The term "C₂-C₆-alkenyl" refers to a straight-chain or branched unsaturated hydrocarbon radical having 2 to 6 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₃-C₈-cycloalkyl" refers to monocyclic saturated hydrocarbon radicals having 3 to 8 carbon ring members such as cyclopropyl (C₃H₅), cyclobutyl, cyclopentyl, cyclohexyl, cycloheptyl or cyclooctyl.

The term "C₃-C₈-cycloalkyl-C₁-C₄-alkyl" refers to a cycloalkyl radical having 3 to 8 carbon atoms (as defined above), which is bonded via a C₁-C₄-alkyl group as defined above.

The term "C₃-C₈-cycloalkyloxy" refers to a cycloalkyl radical having 3 to 8 carbon atoms (as defined above), which is bonded via an oxygen.

The term "C(=O)-(C₁-C₆-alkyl)" refers to a radical which is bonded through the carbon atom of the C(=O) group as indicated by the number valence of the carbon atom, whereas the C(=O) group is further substituted by a C₁-C₆-alkyl radical as defined herein. Likewise, a term such as (C=O)-(C₂-C₆-alkynyloxy) refers to a radical which is attached through the carbon atom of the C(=O) group, whereas the C(=O) group is further substituted by a C₂-C₆-alkynyloxy radical as defined herein.

The terms "C₁-C₆-alkyl-(C=O)-amino" or "C₁-C₆-alkyl-(C=O)-(C₁-C₆-alkylamino)" refer to a C(=O)-(C₁-C₆-alkyl) group bonded through an amino group, which is unsubstitued or substituted by a C₁-C₆-alkyl group, respectively.

The terms "C₁-C₆-alkyl-(C=O)-O-C₁-C₆-alkyl, C₃-C₆-cycloalkyl-(C=O)-O-C₁-C₆-alkyl, C₁-C₆-haloalkyl-(C=O)-O-C₁-C₆-alkyl, C₂-C₆-alkenyl-(C=O)-O-C₁-C₆-alkyl, C₂-C₆-alkynyl-(C=O)-O-C₁-C₆-alkyl, C₁-C₆-alkoxy-(C=O)-O-C₁-C₆-alkyl, C₁-C₆-haloalkoxy-(C=O)-O-C₁-C₆-alkyl, C₂-C₆-alkenyloxy-(C=O)-O-C₁-C₆-alkyl or C₂-C₆-alkynyloxy-(C=O)-O-C₁-C₆-alkyl" refer to radicals which are bonded via the C₁-C₆-alkyl group as defined at the right end of the group expression, for example C₂-C₆-alkynyloxy-(C=O)-O-C₁-C₆-alkyl refers to a carbonate residue, i.e. an alkynyloxy-(C=O)-O- radical, which is bonded via a C₁-C₆-alkyl group as defined above and which attaches to this C₁-C₆-alkyl group through the oxygen atom.

The terms "(C=O)-(phenyl-C₁-C₄-alkyl)" or "(C=O)-(heteroaryl-C₁-C₄-alkyl)" refer to a radical which is attached through the carbon atom of the C(=O) group as indicated by the number valence of the carbon atom, whereas the C(=O) group is further substituted by a phenyl-C₁-C₄-alkyl or heteroaryl-C₁-C₄-alkyl radical as defined herein.

The term "aliphatic" refers to compounds or radicals composed of carbon and hydrogen and which are non-aromatic compounds. An alicyclic compound or radical is an organic compound that is both aliphatic and cyclic. They contain one or more all-carbon rings which may be either saturated or unsaturated, but do not have aromatic character.

The term "cyclic moieties" refers to a radical which is an alicyclic ring or an aromatic ring, such as, for example, phenyl or heteroaryl.

The term "and wherein the aliphatic or cyclic/alicyclic groups are unsubstituted or substituted" refers to aliphatic groups, (ali)cyclic groups and groups, which contain an aliphatic and an (ali)cyclic moiety in one group, such as in, for example, phenyl-C₁-C₄-alkyl; therefore a group which contains an aliphatic and a cyclic moiety both of these moieties may be substituted or unsubstituted independently of each other.

The term "phenyl" refers to an aromatic ring systems incuding six carbon atoms (also referred to as benzene ring).

The term "heteroaryl" refers to aromatic monocyclic or polycyclic ring systems incuding besides carbon atoms, 1, 2, 3 or 4 heteroatoms independently selected from the group consisting of N, O and S.

The term "5- or 6-membered heteroaryl" refers to monocyclic aromatic ring systems including as ring member atoms besides one or more carbon atoms, 1, 2, 3 or 4 heteroatoms independently selected from the group consisting of N, O and S, for example, a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

Further embodiments of the present invention are evident from the claims, the description and the examples. It is to be understood that the features mentioned above and still to be illustrated below of the subject matter of the invention can be applied not only in the combination given in each particular case but also in other combinations, without leaving the scope of the invention. As regards the herbicidal activity of the compounds of the formula (I), and, where applicable, also regarding compounds of all sub-formulae provided herein preference is given to those compounds wherein the substituents and variables such as R^{c}, R^{d}, Re, R^{N}, X, n and m have independently of each other or more preferably in combination (any possible combination of 2 or more substituents as defined herein) the meanings as defined or preferably defined below. The groups mentioned herein for a substituent or for a combination of substituents are furthermore, independently of the combination in which they are mentioned, a particularly preferred embodiment of the substituent or of the combination of substituents in question.

In one preferred embodiment of the invention X is hydrogen, fluorine, chlorine or C₁-C₆-fluoroalkyl. In another preferred embodiment X is hydrogen, fluorine or chlorine. In a further preferred embodiment X is hydrogen or fluorine, particularly X is fluorine.

In one preferred embodiment of the invention R^{N} is hydrogen, C₁-C₆-alkoxy-C₁-C₆-alkyl, propargyl, benzyl, (C=O)-(C₁-C₆-alkyl), (C=O)-(C₁-C₆-haloalkyl), (C=O)-(C₂-C₆-alkynyl), (C=O)-(C₃-C₆-cycloalkyl), (C=O)-(C₁-C₆-alkoxy), (C=O)-(C₁-C₆-haloalkoxy), (C=O)-(C₂-C₆-alkynyloxy), (C=O)-(C₁-C₆-alkylthio), (C=O)-(C₁-C₆-dialkylamino), (C=O)-(phenyl), (C=O)-(heteroaryl), (C=O)-(phenyl-C₁-C₄-alkyl), (C=O)-(heteroaryl-C₁-C₄-alkyl), C₁-C₆-alkylsulfonyl, C₁-C₆-haloalkylsulfonyl, C₃-C₆-cycloalkylsulfonyl, C₁-C₆-dialkylaminosulfonyl, phenylsulfonyl, heteroarylsulfonyl, C₁-C₆-alkyl-carbonyloxy- C₁-C₆-alkyl, C₁-C₆-haloalkyl-carbonyloxy- C₁-C₆-alkyl, C₁-C₆-alkoxy-carbonyloxy- C₁-C₆-alkyl or C₁-C₆-alkynyloxy-carbonyloxy-C₁-C₆-alkyl; and wherein the aliphatic and cyclic moieties of R^{N} are unsubstituted or substituted by 1, 2, 3 or up to the maximum number of identical or different groups R^{N1}; wherein R^{N1} is fluoro, chloro, bromo, cyano, nitro, methyl or methoxy.

In a further preferred embodiment R^{N} is hydrogen, propargyl, C₁-C₆-alkoxy-C₁-C₆-alkyl, (C=O)-(C₁-C₆-alkyl), (C=O)-(C₁-C₆-haloalkyl), (C=O)-(C₃-C₆-cycloalkyl), (C=O)-(C₁-C₆-alkoxy), (C=O)-(C₂-C₆-alkynyloxy), (C=O)-(C₁-C₆-alkylthio), (C=O)-(C₁-C₆-dialkylamino), (C=O)-(phenyl), (C=O)-(heteroaryl), C₁-C₆-alkylsulfonyl, C₁-C₆-haloalkylsulfonyl, C₃-C₆-cycloalkylsulfonyl, phenylsulfonyl, C₁-C₆-alkyl-carbonyloxy- C₁-C₆-alkyl, C₁-C₆-haloalkyl-carbonyloxy- C₁-C₆-alkyl, C₁-C₆-alkoxy-carbonyloxy- C₁-C₆-alkyl or C₁-C₆-alkynyloxy-carbonyloxy-C₁-C₆-alkyl.

In yet another preferred embodiment R^{N} is hydrogen, (C=O)-(C₁-C₆-alkyl), (C=O)-(C₃-C₆-cycloalkyl), (C=O)-(C₁-C₆-alkoxy), (C=O)-(C₂-C₆-alkynyloxy), (C=O)-(C₁-C₆-alkylthio), (C=O)-(phenyl), C₁-C₆-alkylsulfonyl, C₁-C₆-haloalkylsulfonyl, C₃-C₆-cycloalkylsulfonyl, phenylsulfonyl, C₁-C₆-alkyl-carbonyloxy- C₁-C₆-alkyl, C₁-C₆-alkoxy-carbonyloxy- C₁-C₆-alkyl or C₁-C₆-alkynyloxy-carbonyloxy- C₁-C₆-alkyl.

In a further preferred embodiment R^{N} is hydrogen, (C=O)-(C₁-C₆-alkyl), (C=O)-(C₃-C₆-cycloalkyl), (C=O)-(C₁-C₆-alkoxy), (C=O)-(C₂-C₆-alkynyloxy), (C=O)-(phenyl), C₁-C₆-alkyl-carbonyloxy- C₁-C₆-alkyl.

In another preferred embodiment R^{N} is hydrogen, -(C=O)-CH₃, -(C=O)-CH₂CH₃, -(C=O)-CH(CH₃)₂ -(C=O)-CH₂CH₂CH₃, -(C=O)-cyclopropyl, -(C=O)-O-CH₃, -(C=O)-O-(3-chlorophenyl), -(C=O)-O-CH(CH₃)₂, -(C=O)-O-C(CH₃)₃, -(C=O)-O-benzyl -(C=O)-O-propargyl, -S(O)₂-CH₃. -S(O)₂-CF₃, propargyl, -(C=O)-phenyl, -(C=O)-(2-chlorophenyl), -(C=O)-(4-chlorophenyl), -(C=O)-(2-methylphenyl), -(C=O)-(4-methylphenyl), -(C=O)-(3-methylphenyl), -CH(CH₃)-O-(C=O)-CH₃, -CH₂-O(C=O)-C(CH₃)₃ or -CH(CH₃)-O-(C=O)-C(CH₃)₃.

In still another preferred embodiment R^{N} is hydrogen, (C=O)-(C₁-C₆-alkyl) or (C=O)-(C₁-C₆-alkoxy).

In yet another preferred embodiment R^{N} is hydrogen.

Further preferred embodiments of the invention relate to compounds of the formula (I), in which the group R^{N} is selected from the group consisting of radicals RN-1 to RN-15 represented in Table N.

**Table N:**

| **RN-ID** | **R^{N}** |
|---|---|
| RN-1 | hydrogen |
| RN-2 | -(C=O)-CH₃ |
| RN-3 | -(C=O)-CH(CH₃)₂ |
| RN-4 | -(C=O)-cyclopropyl |
| RN-5 | -(C=O)-O-CH₃ |
| RN-6 | -(C=O)-O-CH₂CH₃ |
| RN-7 | -(C=O)-O-CH(CH₃)₂ |
| RN-8 | -(C=O)-O-C(CH₃)₃ |
| RN-9 | -(C=O)-O-propargyl |
| RN-10 | -S(O)₂-CH₃ |
| RN-11 | -S(O)₂-CF₃ |
| RN-12 | -(C=O)-phenyl |
| RN-13 | -CH(CH₃)-O-(C=O)-CH₃ |
| RN-14 | -CH₂-O(C=O)-C(CH₃)₃ |
| RN-15 | -CH(CH₃)-O-(C=O)-C(CH₃)₃ |

In one preferred embodiment R^{c} is independently selected from the group consisting of halogen, cyano, C₁-C₆-alkyl, C₁-C₆-alkoxy, C₁-C₆-haloalkyl, C₁-C₆-haloalkoxy, C₁-C₄-alkoxy-C₁-C₄-alkyl, C₁-C₆-alkyl-(C=O)-amino, C₁-C₆-alkyl-(C=O)-(C₁-C₆-alkylamino), C₂-C₆-alkenyl, C₂-C₆-alkynyl, C₂-C₆-alkenyloxy, C₂-C₆-alkynyloxy, C₃-C₈-cycloalkyl, C₃-C₈-cycloalkoxy, (C=O)-(C₁-C₆-alkyl), (C=O)-(C₁-C₆-alkenyl), (C=O)-(C₁-C₆-alkynyl), (C=O)-(C₁-C₆-alkoxy), (C=O)-(C₁-C₆-alkenyloxy), (C=O)-(C₁-C₆-alkynyloxy), (C=N-O-(C₁-C₆-alkyl))-(C₁-C₆-alkyl), (CH=N-O-(C₁-C₆-alkyl)) and (C=N-O-(C₁-C₆-alkyl))-(C₁-C₆-alkoxy); and wherein the aliphatic and alicyclic moieties are unsubstituted or substituted by 1, 2, 3 or up to the maximum number of identical or different groups R^{c1}; wherein R^{c1} is halogen, cyano, C₁-C₄-alkyl, C₁-C₄-alkoxy, C₁-C₄-alkylthio, C₁-C₄-alkylsulfinyl, C₁-C₄-alkylsulfonyl, C₁-C₄-alkylamino, C₁-C₄-dialkylamino, C₃-C₆-cycloalkyl, C₁-C₄-haloalkyl or C₁-C₄-haloalkoxy.

In another preferred embodiment R^{c} is independently selected from the group consisting of fluorine, chlorine, C₁-C₆-alkyl, C₁-C₆-alkoxy, C₁-C₆-haloalkyl, C₁-C₆-haloalkoxy, C₁-C₆-alkyl-(C=O)-amino, C₁-C₆-alkyl-(C=O)-(C₁-C₆-alkylamino), C₂-C₆-alkenyl, C₂-C₆-alkynyl, C₂-C₆-alk-enyloxy, C₂-C₆-alkynyloxy, C₃-C₈-cycloalkoxy, (C=O)-(C₁-C₆-alkyl), (C=O)-(C₁-C₆-alkoxy) and (C=N-O-(C₁-C₆-alkyl))-(C₁-C₆-alkyl); and wherein the aliphatic and alicyclic moieties are unsubstituted or substituted by 1, 2, 3 or up to the maximum number of identical or different groups R^{c1}; wherein R^{c1} is fluorine, C₁-C₄-alkyl, C₁-C₄-alkoxy, C₃-C₆-cycloalkyl, C₁-C₄-haloalkyl or C₁-C₄-haloalkoxy.

In a further preferred embodiment R_{c} is independently selected from the group consisting of fluorine, C₁-C₆-alkyl, C₁-C₆-alkoxy, C₁-C₆-haloalkyl, C₁-C₆-haloalkoxy, C₂-C₆-alkenyl, C₂-C₆-alkynyl, C₂-C₆-alkenyloxy, C₂-C₆-alkynyloxy, (C=O)-(C₁-C₆-alkyl), (C=O)-(C₁-C₆-alkoxy) and (C=N-O-(C₁-C₆-alkyl))-(C₁-C₆-alkyl); and wherein the aliphatic and alicyclic moieties are unsubstituted or substituted by 1, 2, 3 or up to the maximum number of identical or different groups R^{c1}; wherein R^{c1} is fluorine, C₁-C₄-alkyl, C₁-C₄-alkoxy, C₁-C₄-haloalkyl or C₁-C₄-haloalkoxy.

In still another preferred embodiment R^{c} is independently selected from the group consisting of fluorine, C₁-C₆-alkyl, C₁-C₆-alkoxy, C₁-C₆-haloalkyl, C₁-C₆-haloalkoxy, C₂-C₆-alkenyl, C₂-C₆-alkynyl, (C=O)-(C₁-C₆-alkyl), (C=O)-(C₁-C₆-alkoxy) and (C=N-O-(C₁-C₆-alkyl))-(C₁-C₆-alkyl).

In a further preferred embodiment R^{c} is independently selected from the group consisting of fluorine, C₁-C₆-alkyl, C₁-C₆-alkoxy, C₁-C₆-haloalkyl, C₁-C₆-haloalkoxy, C₂-C₆-alkynyl and C₁-C₄-alkoxy-C₁-C₄-alkyl.

In another preferred embodiment R^{c} is independently selected from the group consisting of fluorine, methyl, ethyl, propyl, allyl, vinyl, ethynyl, propargyl, CHF₂, CF₃, OCH₃, OCHF₂ and OCF₃.

In still another preferred embodiment R^{c} is independently selected from the group consisting of fluorine, methyl, -(C=O)CH₃ or -(C=O)OCH₃.

In a preferred embodiment n is 0, 1 or 2, particularly 0 or 1. In another preferred embodiment n is 2. In a further preferred embodiment n is 1. Most preferably n is 0.

In a preferred embodiment Re is independently selected from the group consisting of halogen, cyano, OH, SH, NH₂, C₁-C₆-alkyl, C₁-C₆-haloalkyl, C₁-C₆-haloalkoxy, C₁-C₆-alkoxy, C₁-C₆-alkoxy-C₁-C₆-alkyl, C₁-C₆-alkylthio, C₁-C₆-alkylsulfinyl, C₁-C₆-alkylsulfonyl, C₁-C₄-alkylamino and C₁-C₄-dialkylamino. In another preferred embodiment Re is independently selected from the group consisting of halogen, cyano, C₁-C₆-alkyl, C₁-C₆-haloalkyl, C₁-C₆-alkoxy, C₁-C₆-haloalkoxy and C₁-C₄-dialkylamino. In another preferred embodiment R^{e} is independently selected from the group consisting of halogen, C₁-C₆-alkyl, C₁-C₆-haloalkyl, C₁-C₆-alkoxy and C₁-C₄-haloalkoxy.

In a further preferred embodiment Re is independently selected from the group consisting of fluorine, chlorine, methyl, methoxy, trifluoromethyl and difluoromethyl, trifluoromethoxy and difluoromethoxy; most preferably Re is fluorine.

In a preferred embodiment m is 0 or 1, particularly 0.

In one aspect of the invention R^{d} is fluorine, chlorine or bromine. In another aspect of the invention R^{d} is fluorine or chlorine, particularly fluorine. In a further aspect of the invention R^{d} is chlorine. In yet another aspect of the invention R^{d} is bromine.

In one preferred embodiment the present invention relates to compounds of the formula (I) or their N-oxides or their salts or acid addition salts, wherein
- X: is fluorine;
- R^{N}: is hydrogen, C₁-C₆-alkyl, C₁-C₆-alkoxy-C₁-C₆-alkyl, C₂-C₆-alkenyl, C₂-C₆-alkynyl, C₃-C₆-cycloalkyl, phenyl-C₁-C₄-alkyl, heteroaryl-C₁-C₄-alkyl, (C=O)-(C₁-C₆-alkyl), (C=O)- C₂-C₆-alkynyl, (C=O)-(C₃-C₆-cycloalkyl), (C=O)-(C₁-C₆-alkoxy), (C=O)-(C₂-C₆-alkynyloxy), (C=O)-(C₁-C₆-alkylthio), (C=O)-(C₁-C₆-alkylamino), (C=O)-(C₁-C₆-dialkylamino), (C=O)-phenyl, (C=O)-heteroaryl, (C=O)-(phenyl-C₁-C₄-alkyl), (C=O)-(heteroaryl-C₁-C₄-alkyl), C₁-C₆-alkylsulfonyl, C₃-C₆-cycloalkylsulfonyl, C₁-C₆-alkoxysulfonyl, C₁-C₆-alkylaminosulfonyl, C₁-C₆-dialkylaminosulfonyl, phenylsulfonyl, heteroarylsulfonyl, C₁-C₆-alkyl-(C=O)-O-C₁-C₆-alkyl, C₃-C₆-cycloalkyl-(C=O)-O-C₁-C₆-alkyl, C₁-C₆-haloalkyl-(C=O)-O-C₁-C₆-alkyl, C₂-C₆-alkenyl-(C=O)-O-C₁-C₆-alkyl, C₂-C₆-alkynyl-(C=O)-O-C₁-C₆-alkyl, C₁-C₆-alkoxy-(C=O)-O-C₁-C₆-alkyl, C₁-C₆-haloalkoxy-(C=O)-O-C₁-C₆-alkyl, C₂-C₆-alkenyloxy-(C=O)-O-C₁-C₆-alkyl or C₂-C₆-alkynyloxy-(C=O)-O-C₁-C₆-alkyl; and wherein the aliphatic and cyclic moieties of R^{N} are unsubstituted or substituted by 1, 2, 3 or up to the maximum number of identical or different groups R^{N1}; wherein
R^{N1} is halogen, hydroxy, cyano, nitro, C₁-C₄-alkyl, C₁-C₄-alkoxy, C₁-C₄-alkylthio, C₁-C₄-alkylsulfinyl, C₁-C₄-alkylsulfonyl, C₁-C₄-alkylamino, C₁-C₄-dialkylamino, C₃-C₆-cycloalkyl, C₁-C₄-haloalkyl or C₁-C₄-haloalkoxy;
- R^{c}: is fluoro, C₁-C₆-alkyl, C₁-C₆-alkoxy, C₁-C₆-haloalkyl, C₁-C₆-haloalkoxy, C₂-C₆-alkenyl, C₂-C₆-alkynyl, C₂-C₆-alkenyloxy, C₂-C₆-alkynyloxy, (C=O)-(C₁-C₆-alkyl), (C=O)-(C₁-C₆-alkoxy) or (C=N-O-(C₁-C₆-alkyl))-(C₁-C₆-alkyl); and wherein the aliphatic and alicyclic moieties are unsubstituted or substituted by 1, 2, 3 or up to the maximum number of identical or different groups R^{c1}; wherein R^{c1} is fluoro, C₁-C₄-alkyl, C₁-C₄-alkoxy, C₁-C₄-haloalkyl or C₁-C₄-haloalkoxy;
- n: is 0 or 1;
- m: is 0;
- R^{d}: is fluorine, chlorine or bromine.

In one preferred embodiment the present invention relates to compounds of the formula (I) or their N-oxides or their salts or acid addition salts, wherein
- X: is fluorine;
- R^{N}: is hydrogen, C₁-C₆-alkyl, C₁-C₆-alkoxy-C₁-C₆-alkyl, C₂-C₆-alkenyl, C₂-C₆-alkynyl, C₃-C₆-cycloalkyl, phenyl-C₁-C₄-alkyl, heteroaryl-C₁-C₄-alkyl, (C=O)-(C₁-C₆-alkyl), (C=O)- C₂-C₆-alkynyl, (C=O)-(C₃-C₆-cycloalkyl), (C=O)-(C₁-C₆-alkoxy), (C=O)-(C₂-C₆-alkynyloxy), (C=O-(C₁-C₆-alkylthio), (C=O)-(C₁-C₆-alkylamino), (C=O)-(C₁-C₆-dialkylamino), (C=O)-phenyl, (C=O)-heteroaryl, (C=O)-(phenyl-C₁-C₄-alkyl), (C=O)-(heteroaryl-C₁-C₄-alkyl), C₁-C₆-alkylsulfonyl, C₃-C₆-cycloalkylsulfonyl, C₁-C₆-alkoxysulfonyl, C₁-C₆-alkylaminosulfonyl, C₁-C₆-dialkylaminosulfonyl, phenylsulfonyl, heteroarylsulfonyl, C₁-C₆-alkyl-(C=O)-O-C₁-C₆-alkyl, C₃-C₆-cycloalkyl-(C=O)-O-C₁-C₆-alkyl, C₁-C₆-haloalkyl-(C=O)-O-C₁-C₆-alkyl, C₂-C₆-alkenyl-(C=O)-O-C₁-C₆-alkyl, C₂-C₆-alkynyl-(C=O)-O-C₁-C₆-alkyl, C₁-C₆-alkoxy-(C=O)-O-C₁-C₆-alkyl, C₁-C₆-haloalkoxy-(C=O)-O-C₁-C₆-alkyl, C₂-C₆-alkenyloxy-(C=O)-O-C₁-C₆-alkyl or C₂-C₆-alkynyloxy-(C=O)-O-C₁-C₆-alkyl; and wherein the aliphatic and cyclic moieties of R^{N} are unsubstituted or substituted by 1, 2, 3 or up to the maximum number of identical or different groups R^{N1}; wherein
R^{N1} is halogen, hydroxy, cyano, nitro, C₁-C₄-alkyl, C₁-C₄-alkoxy, C₁-C₄-alkylthio, C₁-C₄-alkylsulfinyl, C₁-C₄-alkylsulfonyl, C₁-C₄-alkylamino, C₁-C₄-dialkylamino, C₃-C₆-cycloalkyl, C₁-C₄-haloalkyl or C₁-C₄-haloalkoxy;
- R^{c}: is fluoro, C₁-C₆-alkyl, C₁-C₆-alkoxy, C₁-C₆-haloalkyl, C₁-C₆-haloalkoxy, C₂-C₆-alkenyl, C₂-C₆-alkynyl, (C=O)-(C₁-C₆-alkyl), (C=O)-(C₁-C₆-alkoxy) or (C=N-O-(C₁-C₆-alkyl))-(C₁-C₆-alkyl);
- n: is 0 or 1 ;
- m: is 0;
- R^{d}: is fluorine, chlorine or bromine.

In another preferred embodiment the invention relates to compounds of the formula (I.a), wherein the meaning of R^{c1}, R^{c2}, R^{c3} and R^{c4} is defined as in subformulae (I.a1) to (I.a20) in table A below, or their N-oxides or their salts or acid addition salts, and wherein R^{N} and R^{d} are as defined or preferably defined herein.

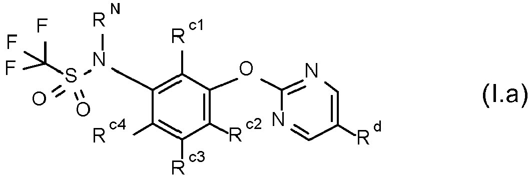

**Table A**

| Subformula | R^{c1} | R^{c2} | R^{c3} | R^{c4} |
|---|---|---|---|---|
| I.a1 | fluorine | hydrogen | hydrogen | hydrogen |
| I.a2 | methyl | hydrogen | hydrogen | hydrogen |
| I.a3 | CO₂CH₃ | hydrogen | hydrogen | hydrogen |
| I.a4 | COCH₃ | hydrogen | hydrogen | hydrogen |
| I.a5 | hydrogen | fluorine | hydrogen | hydrogen |
| I.a6 | hydrogen | methyl | hydrogen | hydrogen |
| I.a7 | hydrogen | CO₂CH₃ | hydrogen | hydrogen |
| I.a8 | hydrogen | COCH₃ | hydrogen | hydrogen |
| I.a9 | hydrogen | hydrogen | fluorine | hydrogen |
| I.a10 | hydrogen | hydrogen | methyl | hydrogen |
| I.a11 | hydrogen | hydrogen | CO₂CH₃ | hydrogen |
| I.a12 | hydrogen | hydrogen | COCH₃ | hydrogen |
| I.a13 | hydrogen | hydrogen | hydrogen | fluorine |
| I.a14 | hydrogen | hydrogen | hydrogen | methyl |
| I.a15 | hydrogen | hydrogen | hydrogen | CO₂CH₃ |
| I.a16 | hydrogen | hydrogen | hydrogen | COCH₃ |
| I.a17 | hydrogen | hydrogen | hydrogen | hydrogen |
| I.a18 | methyl | hydrogen | hydrogen | methyl |
| I.a19 | methyl | methyl | hydrogen | hydrogen |
| I.a20 | hydrogen | methyl | hydrogen | methyl |

In another preferred embodiment the invention relates to compounds of the subformulae (I.a1) to (I.a20) or their N-oxides or their salts or acid addition salts, wherein
- R^{N}: is hydrogen, (C=O)-(C₁-C₆-alkyl), (C=O)-(C₃-C₆-cycloalkyl), (C=O)-(C₁-C₆-alkoxy), (C=O)-(C₂-C₆-alkynyloxy), (C=O)-(C₁-C₆-alkylthio), (C=O)-(phenyl), C₁-C₆-alkylsulfonyl, C₁-C₆-haloalkylsulfonyl, C₃-C₆-cycloalkylsulfonyl, phenylsulfonyl, C₁-C₆-alkyl-carbonyloxy-C₁-C₆-alkyl, C₁-C₆-alkoxy-carbonyloxy- C₁-C₆-alkyl or C₁-C₆-alkynyloxy-carbonyloxy-C₁-C₆-alkyl;
- R^{d}: is fluorine, chlorine or bromine.

In another preferred embodiment the invention relates to compounds of the subformulae (I.a1) to (I.a20) or their N-oxides or their salts or acid addition salts, wherein
- R^{N}: is hydrogen, (C=O)-(C₁-C₆-alkyl), (C=O)-(C₃-C₆-cycloalkyl), (C=O)-(C₁-C₆-alkoxy), (C=O)-(C₂-C₆-alkynyloxy), (C=O)-(phenyl), C₁-C₆-alkyl-(C=O)-O-C₁-C₆-alkyl;
- R^{d}: is fluorine, chlorine or bromine.

In one further preferred embodiment the invention relates to compounds I-1 to I-900 of the formula (I) as defined in Table I below, wherein for each individual compound the respective subformula is selected from subformulae (I.a1) to (I.a20) and the meaning of R^{N} and R^{d} is defined for each compound in one line of Table I, and wherein the groups or variables X, R^{c}, Re, n and m in each case are defined as specified in subformulae (I.a1) to (I.a20) respectively, and wherein for the meaning of R^{N} reference is made to the meaning as defined in Table N for residues RN-1 to RN-15 herein.

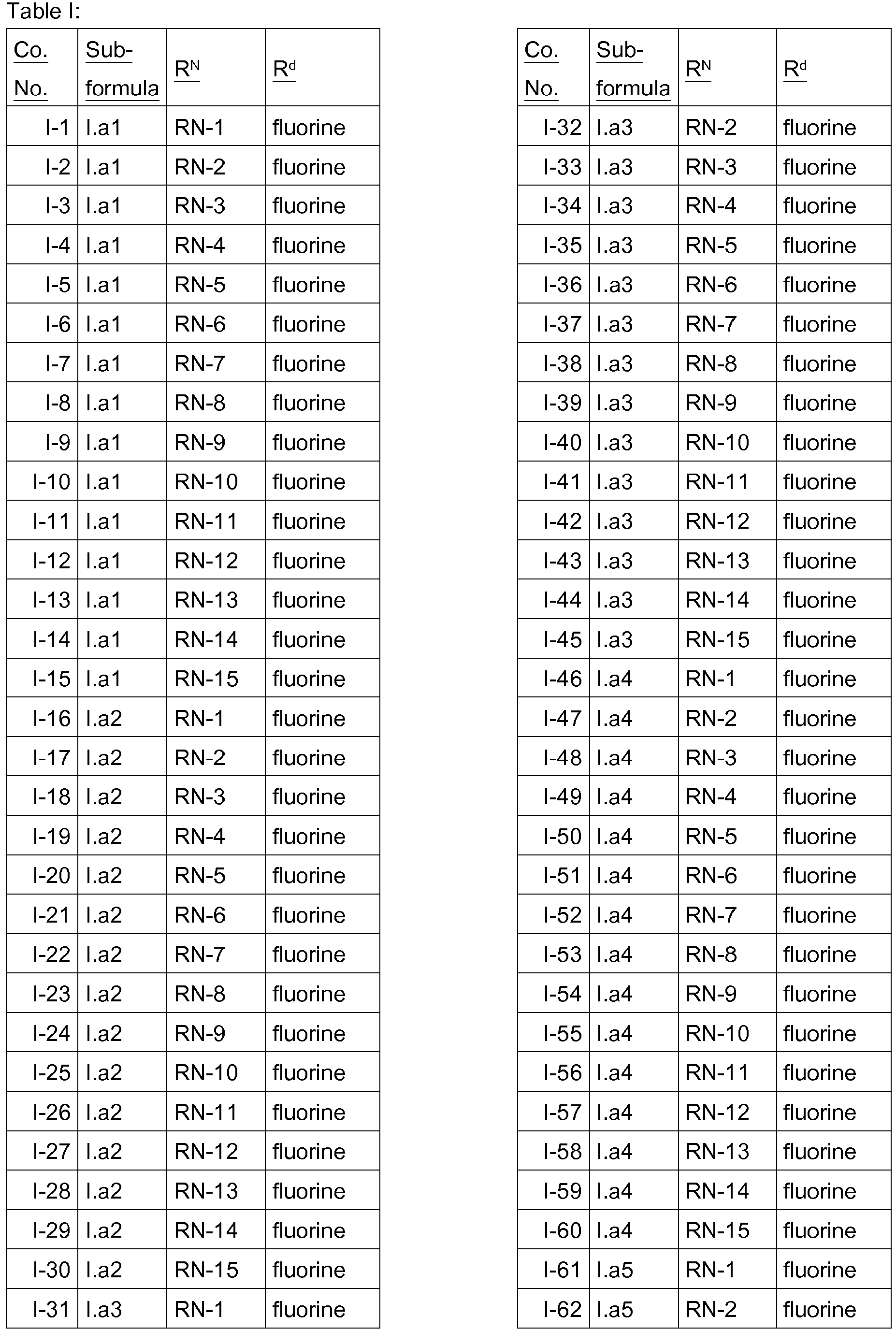

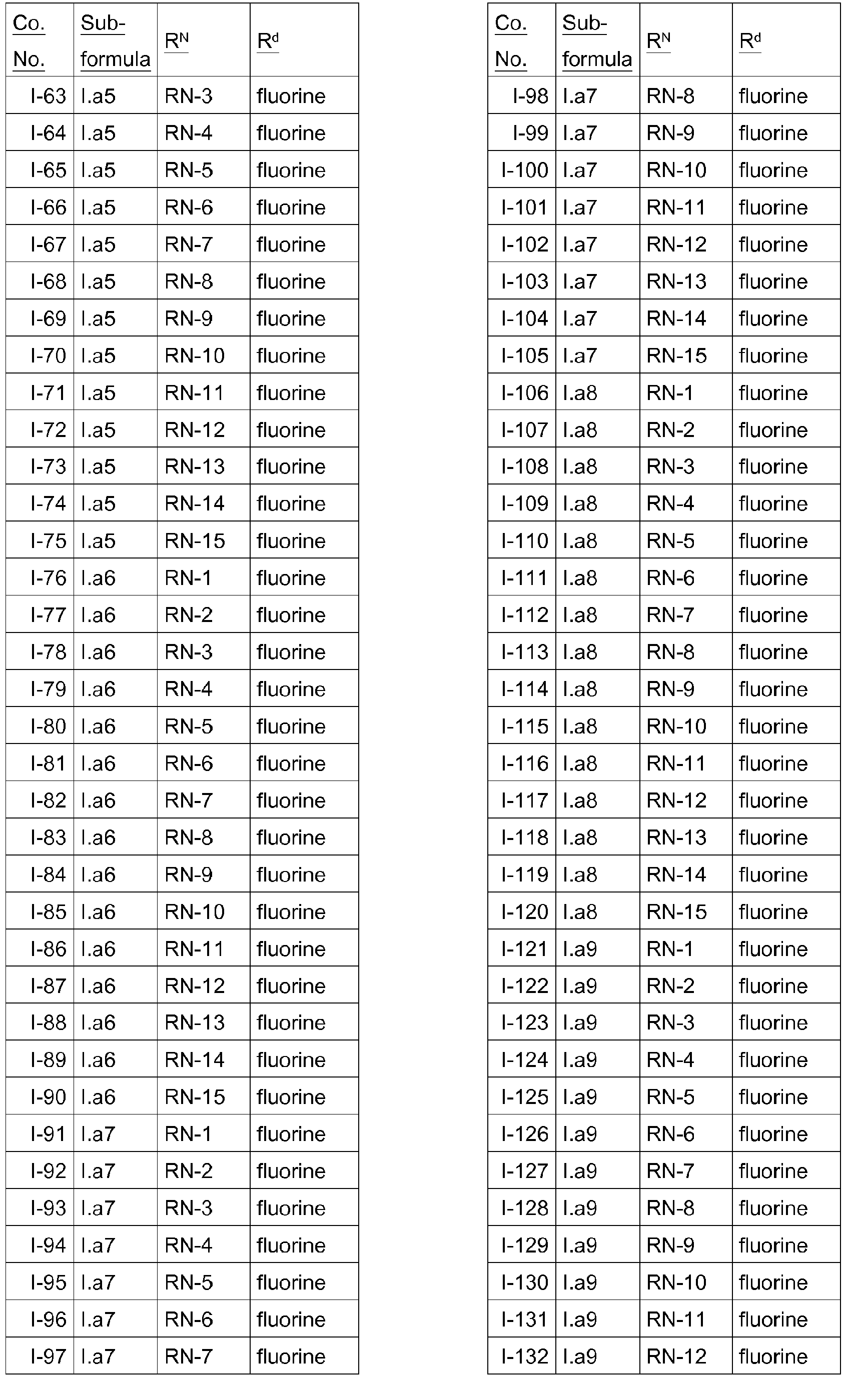

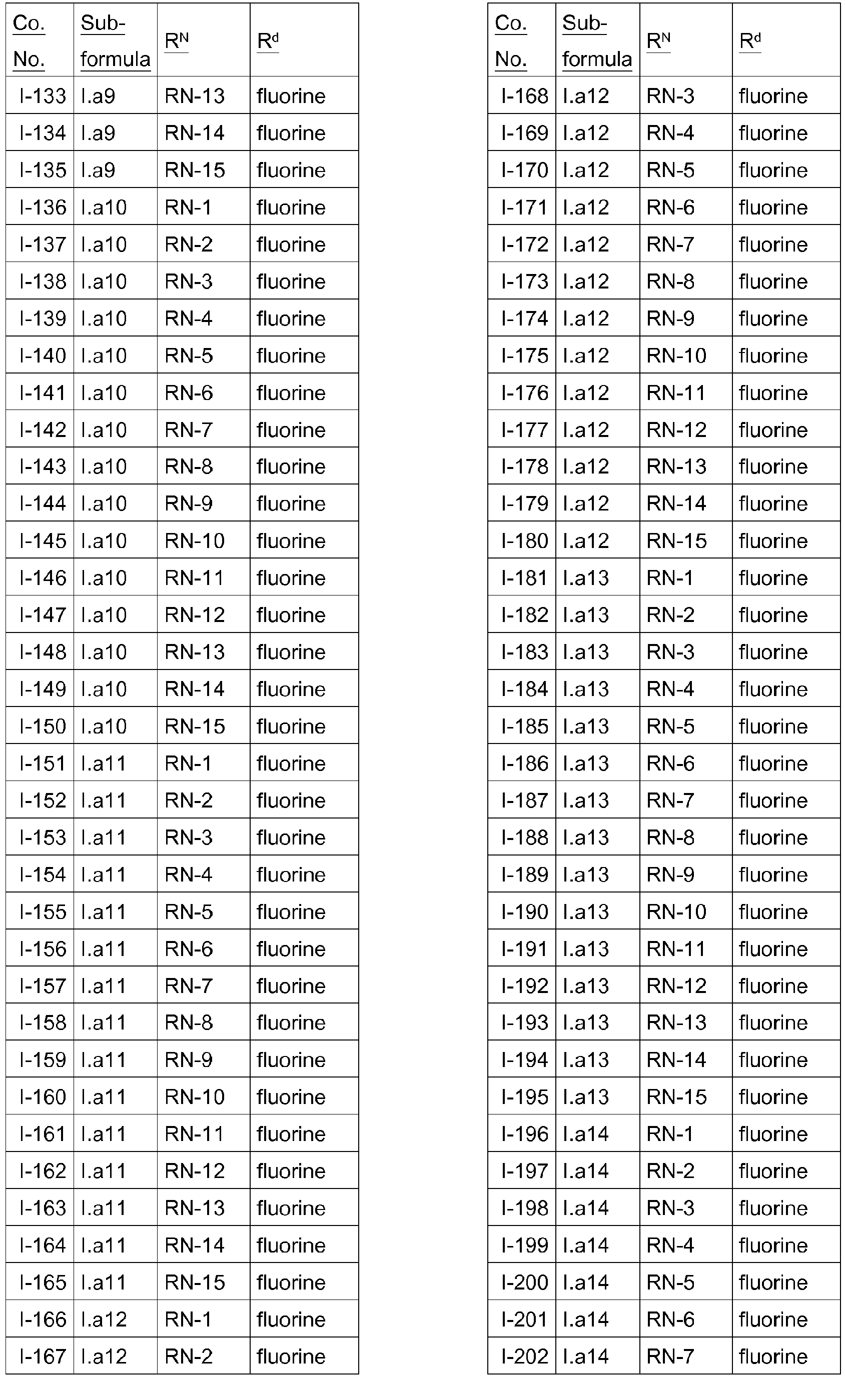

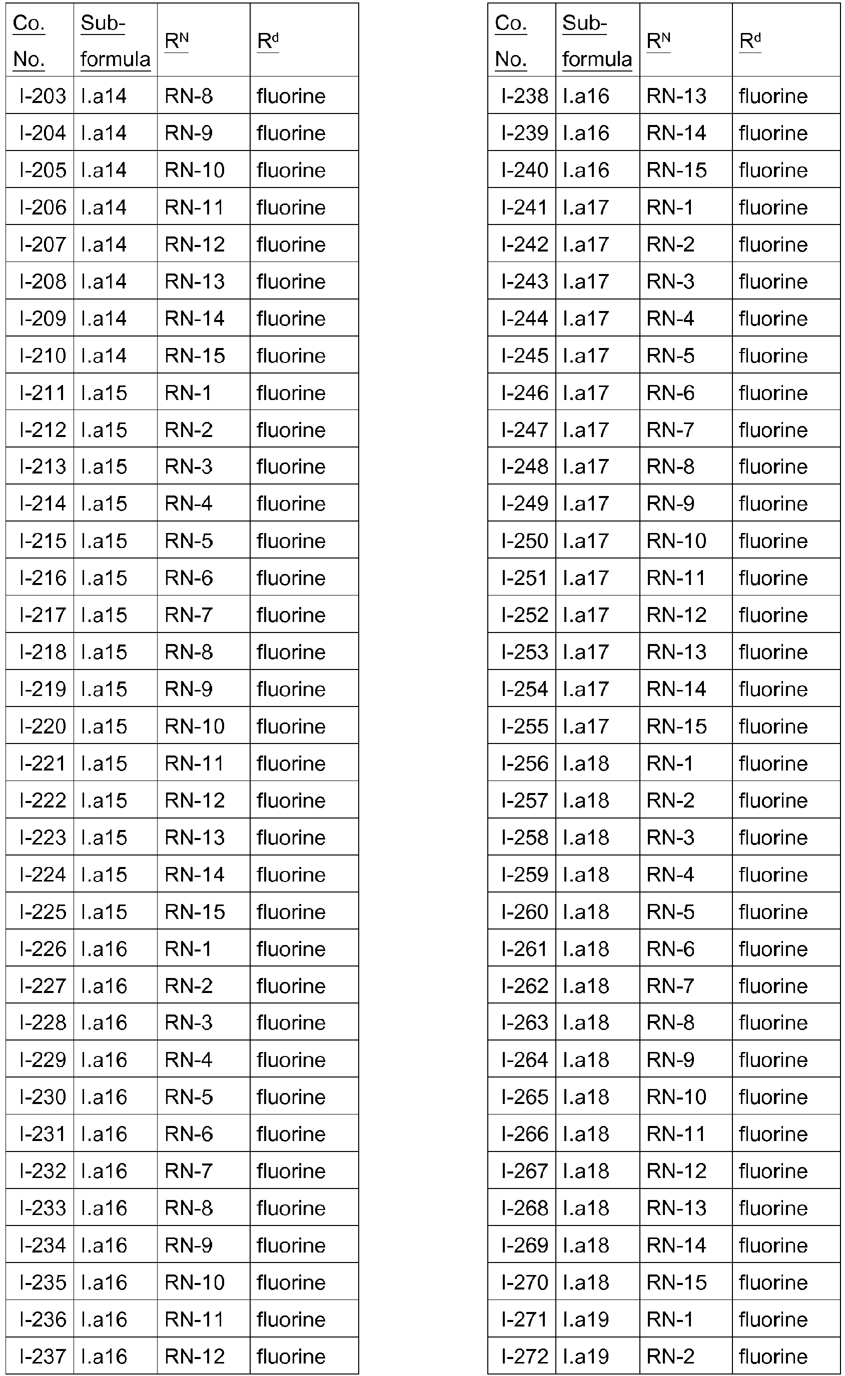

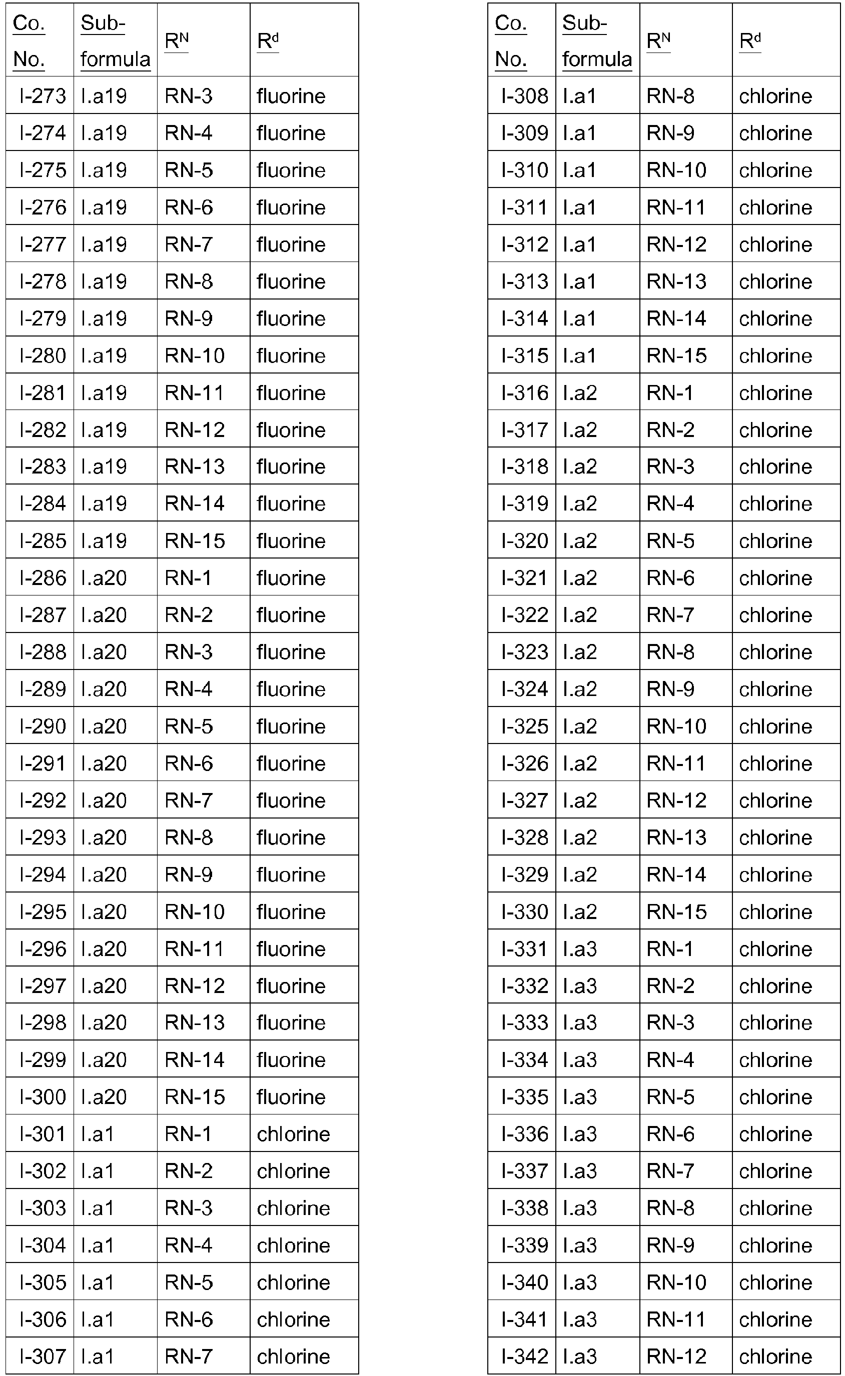

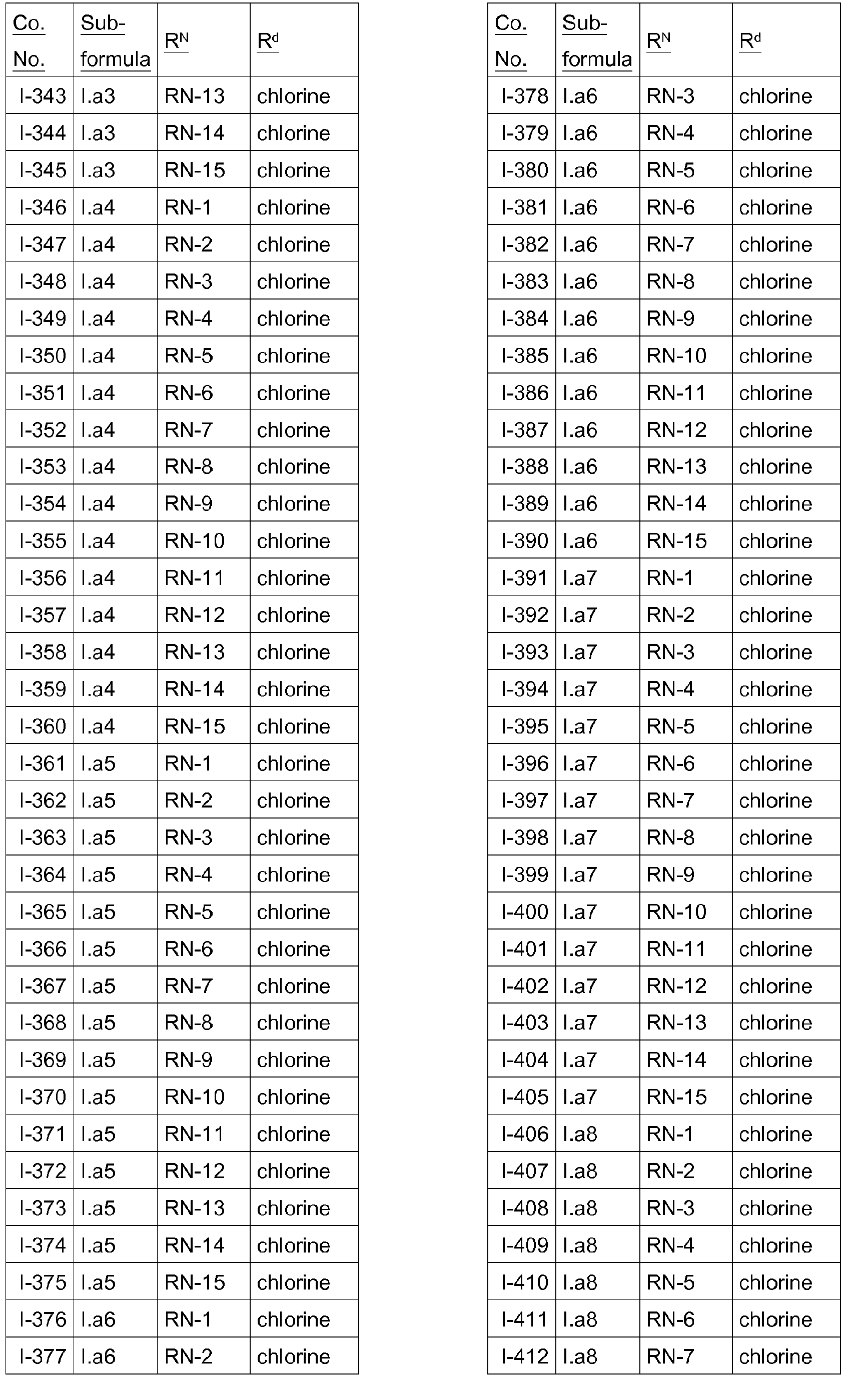

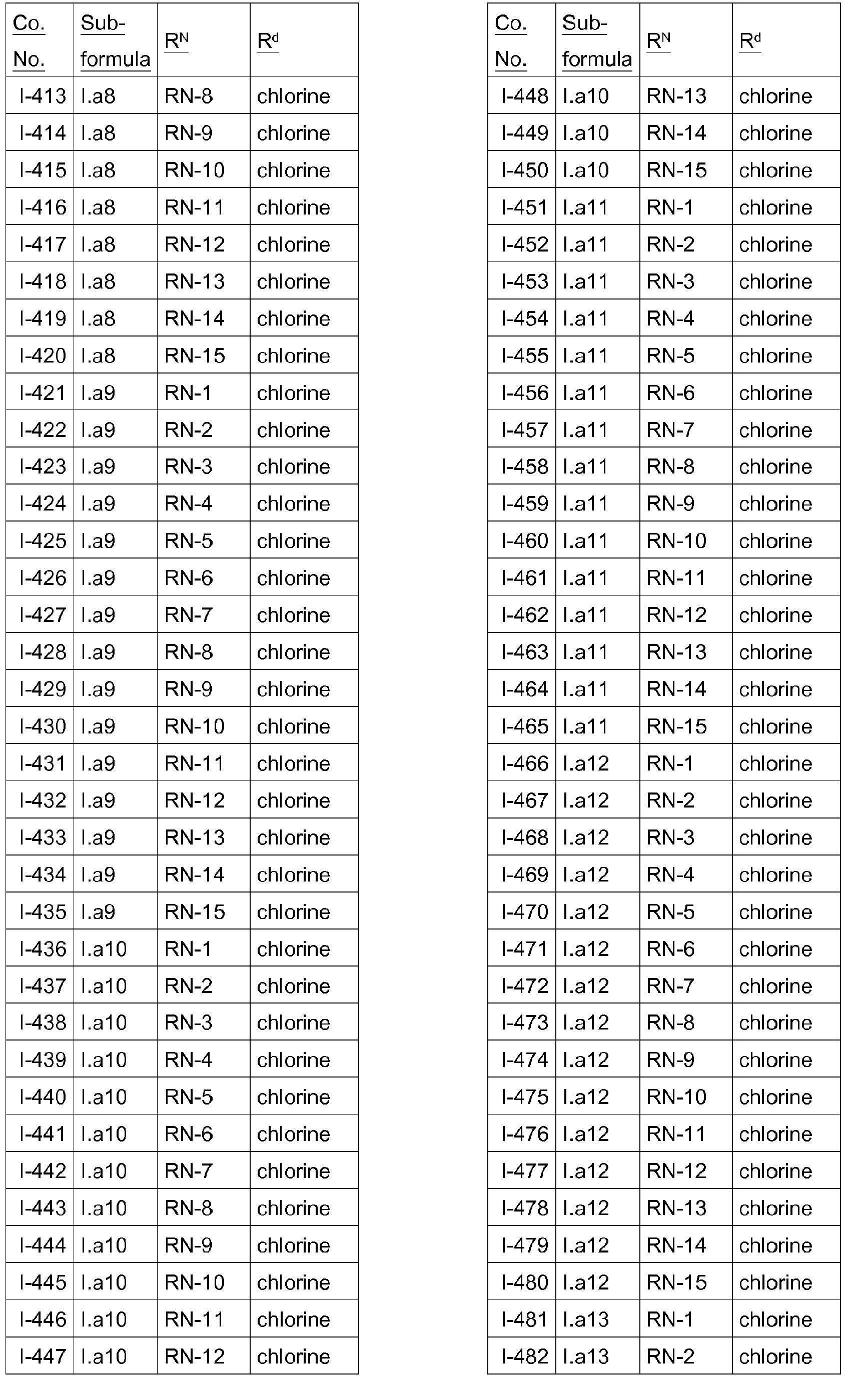

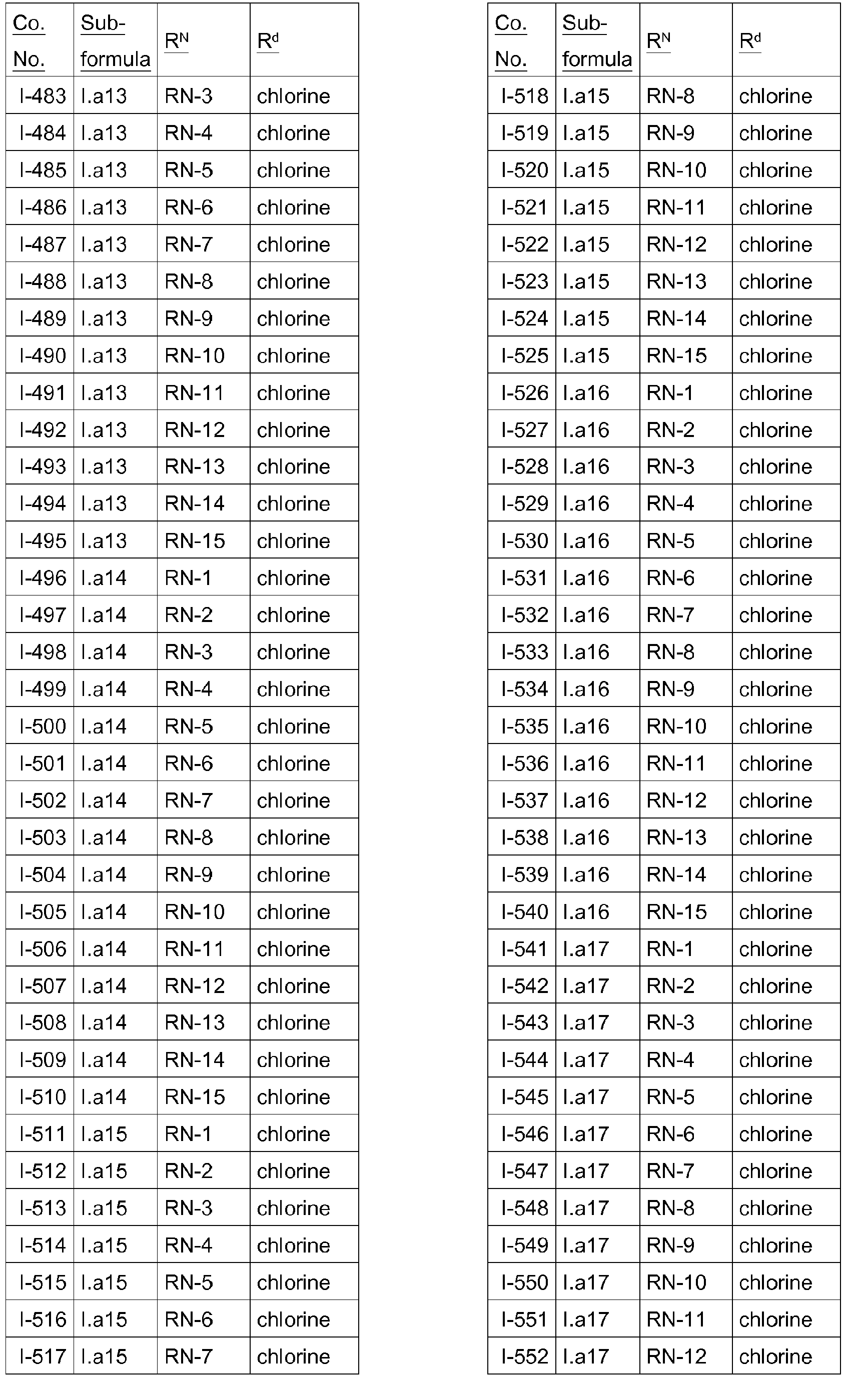

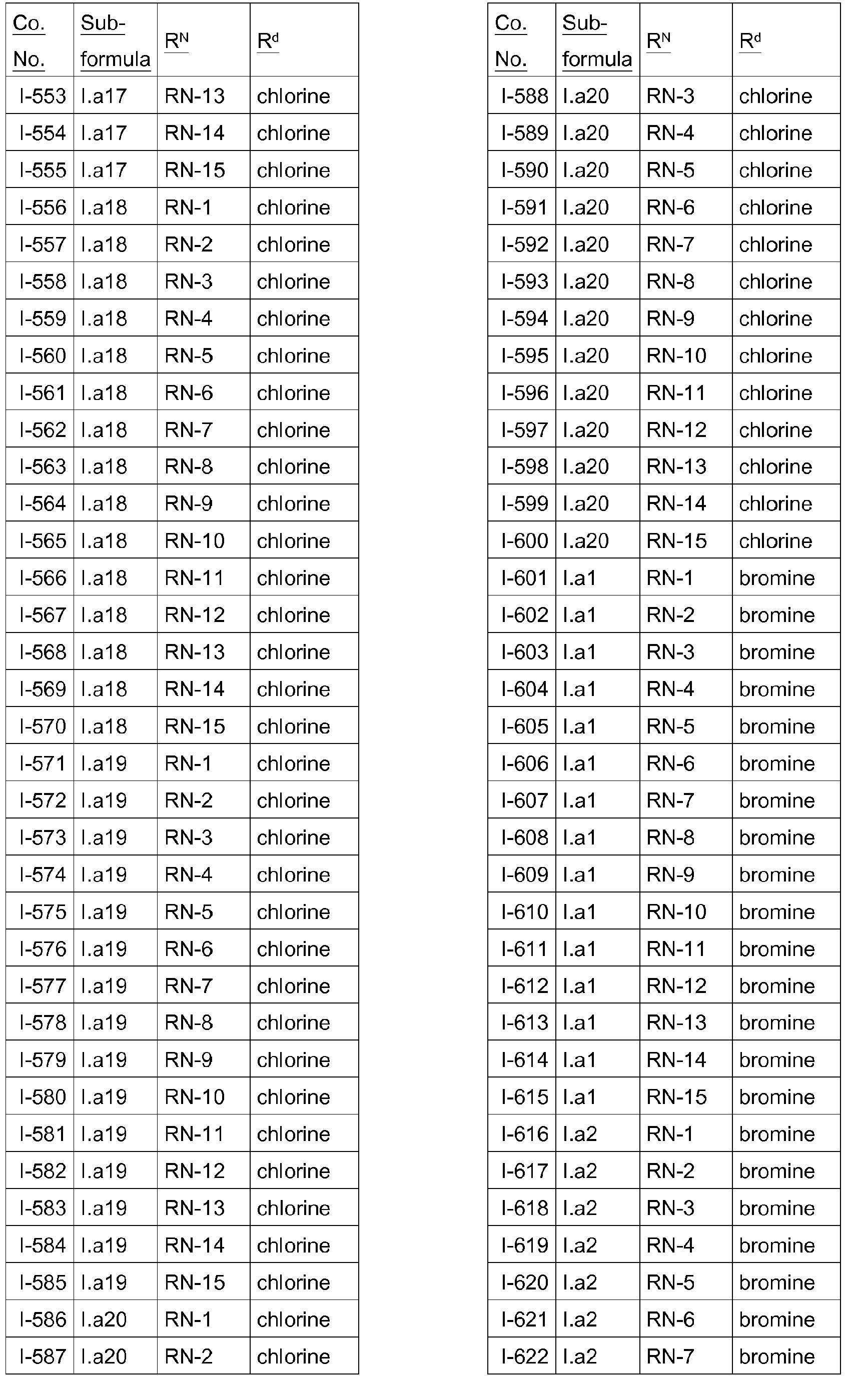

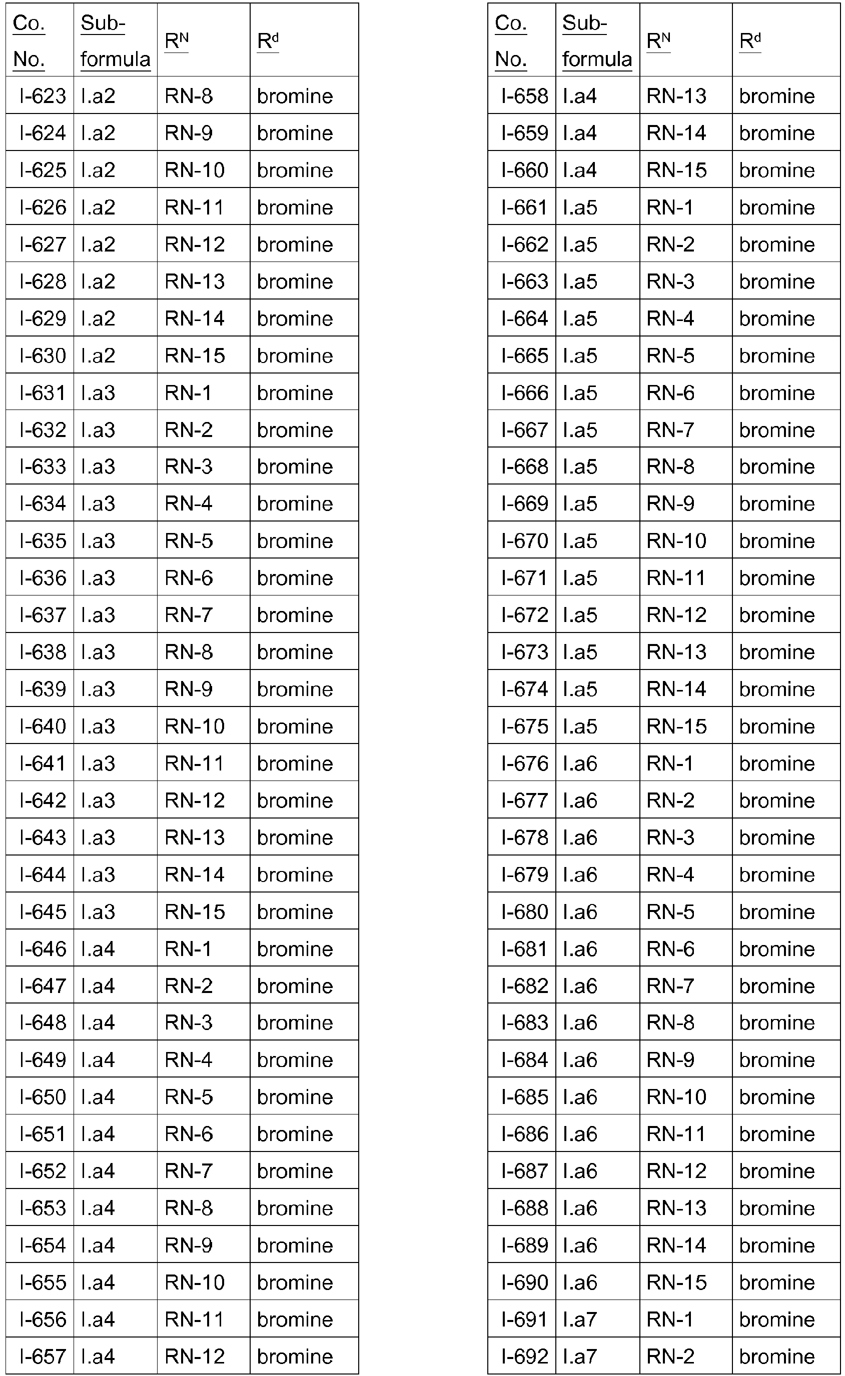

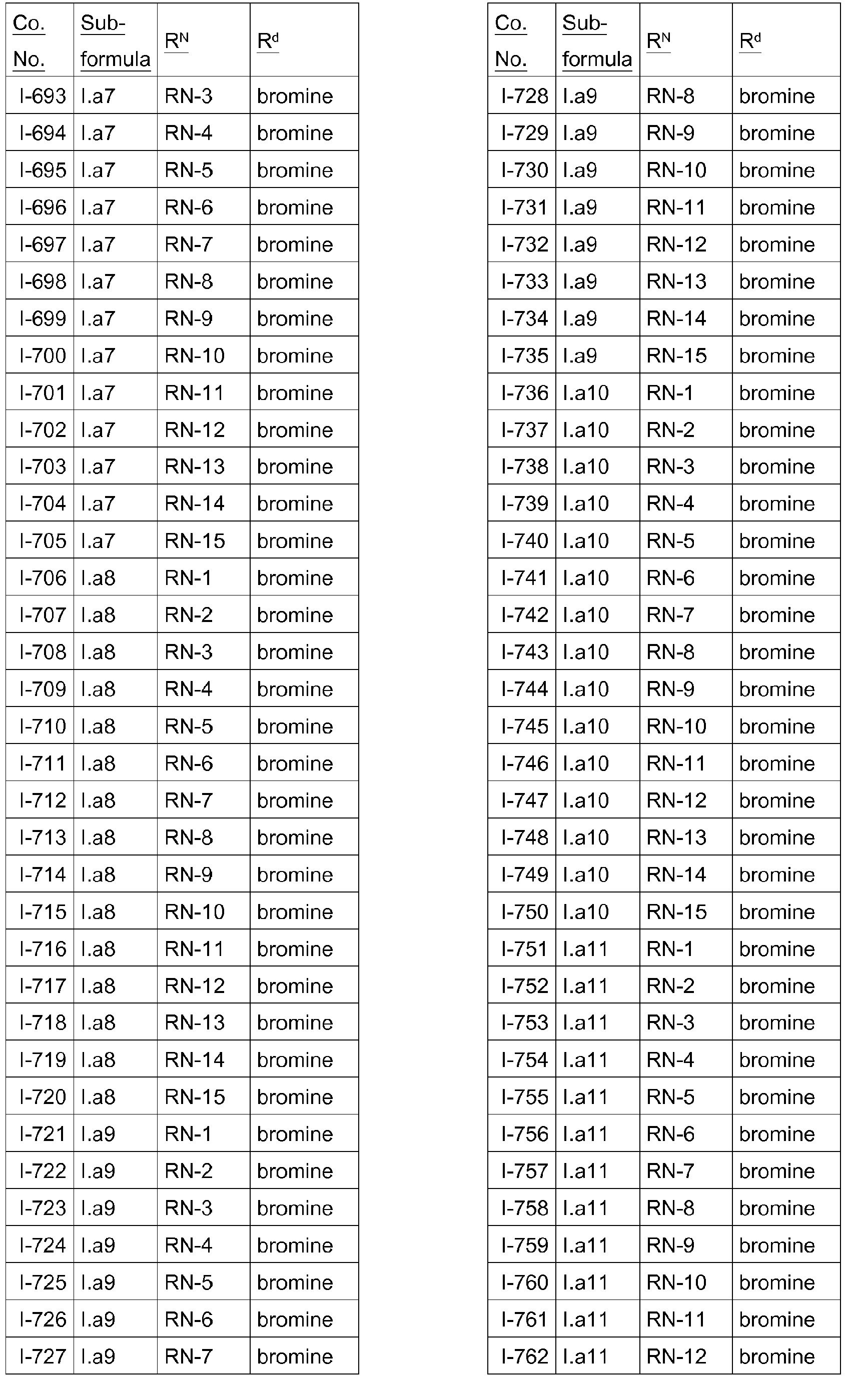

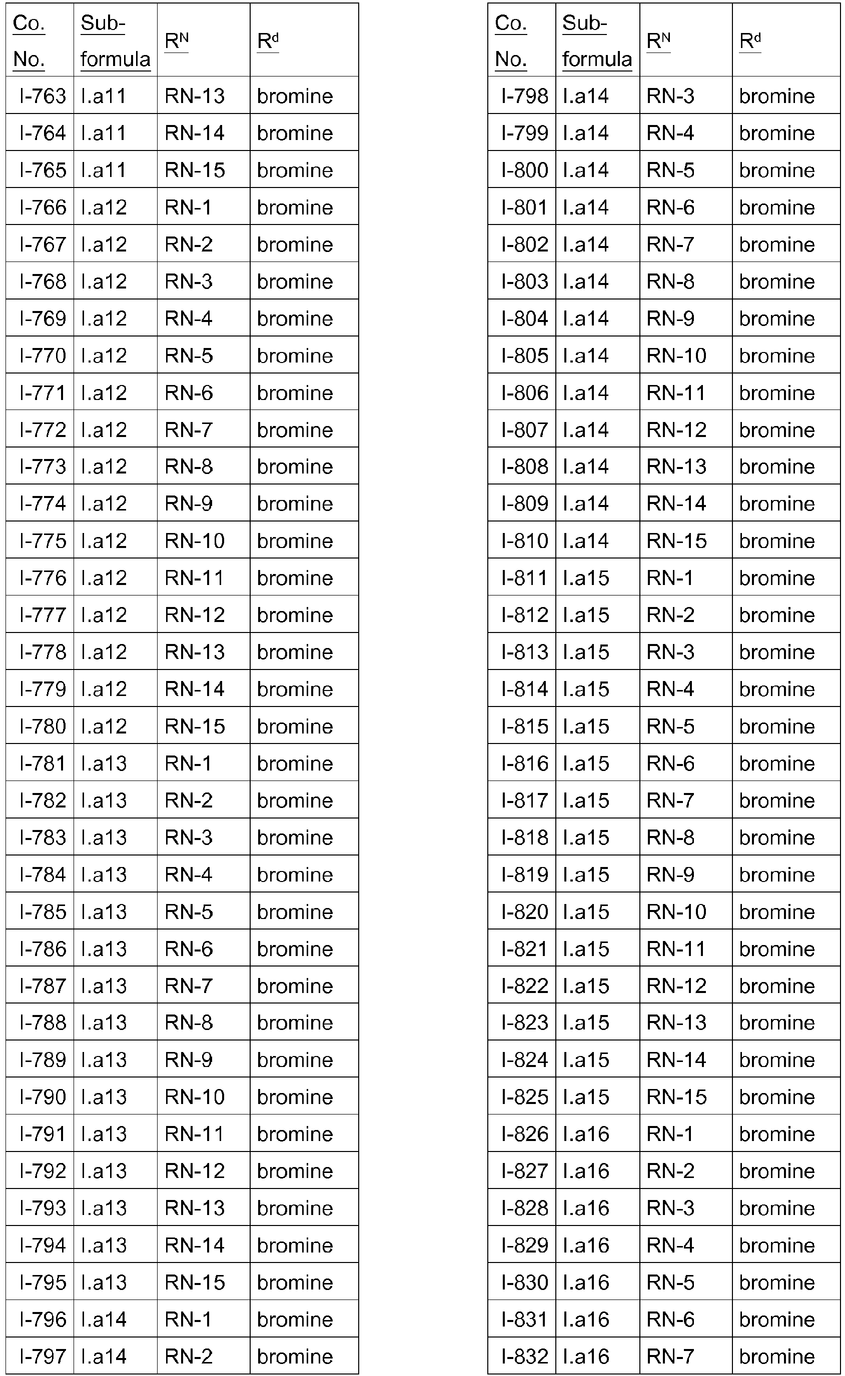

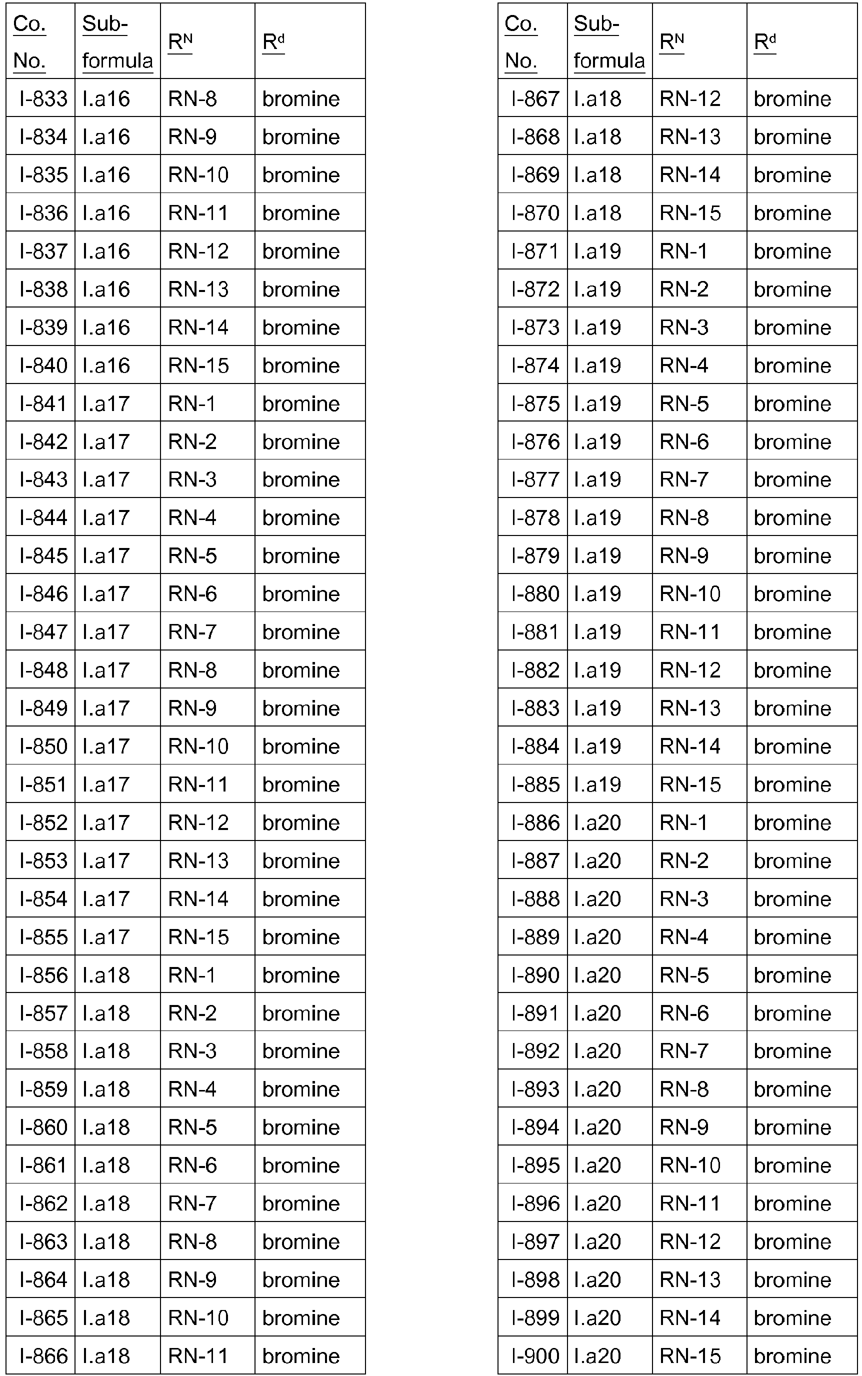

The compounds of the formula (I) can be prepared according to methods or in analogy to methods described in US 435077, US 435076, JP 8193011 and JP 8291146 or following procedures as described herein. The synthesis takes advantage of readily available starting materials that are known, commercially available or may be prepared according to conventional procedures starting from known compounds.

The invention also relates to agrochemical compositions comprising at least one auxiliary and at least one compound of the formula (I) according to the invention.

An agrochemical composition comprises a pesticidally effective amount of a compound of the formula (I). The term "effective amount" denotes an amount of the composition or of the compounds I, which is sufficient for controlling unwanted plants, especially for controlling unwanted plants in cultivated plants and which does not result in a substantial damage to the treated plants. Such an amount can vary in a broad range and is dependent on various factors, such as the plants to be controlled, the treated cultivated plant or material, the climatic conditions and the specific compound of the formula (I) used.

The compounds of the formula (I), their N-oxides, salts or derivatives can be converted into customary types of agrochemical compositions, e. g. solutions, emulsions, suspensions, dusts, powders, pastes, granules, pressings, capsules, and mixtures thereof. Examples for agrochemical composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further agrochemical compositions types are defined in the "Catalogue of pesticide formulation types and international coding system", Technical Monograph No. 2, 6th Ed. May 2008, CropLife International.

The agrochemical compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof. Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof. Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers+Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compounds of the formula (I) on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates. Suitable bactericides are bronopol and isothiazolinone derivatives such as alkylisothiazolinones and benzisothiazolinones.

Suitable anti-freezing agents are ethylene glycol, propylene glycol, urea and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants). Suitable tackifiers or binders are polyvinylpyrrolidons, polyvinylacetates, polyvinyl alcohols, polyacrylates, biological or synthetic waxes, and cellulose ethers.

Examples for composition types and their preparation are:

### i) Water-soluble concentrates (SL, LS)

10-60 wt% of a compound I and 5-15 wt% wetting agent (e. g. alcohol alkoxylates) are dissolved in water and/or in a water-soluble solvent (e. g. alcohols) ad 100 wt%. The active substance dissolves upon dilution with water.

### ii) Dispersible concentrates (DC)

5-25 wt% of a compound I and 1-10 wt% dispersant (e. g. polyvinyl pyrrolidone) are dissolved in organic solvent (e. g. cyclohexanone) ad 100 wt%. Dilution with water gives a dispersion.

### iii) Emulsifiable concentrates (EC)

15-70 wt% of a compound I and 5-10 wt% emulsifiers (e. g. calcium dodecylbenzenesulfonate and castor oil ethoxylate) are dissolved in water-insoluble organic solvent (e. g. aromatic hydrocarbon) ad 100 wt%. Dilution with water gives an emulsion.

### iv) Emulsions (EW, EO, ES)

5-40 wt% of a compound I and 1-10 wt% emulsifiers (e. g. calcium dodecylbenzenesulfonate and castor oil ethoxylate) are dissolved in 20-40 wt% water-insoluble organic solvent (e. g. aromatic hydrocarbon). This mixture is introduced into water ad 100 wt% by means of an emulsifying machine and made into a homogeneous emulsion. Dilution with water gives an emulsion.

### v) Suspensions (SC, OD, FS)

In an agitated ball mill, 20-60 wt% of a compound I are comminuted with addition of 2-10 wt% dispersants and wetting agents (e. g. sodium lignosulfonate and alcohol ethoxylate), 0.1-2 wt% thickener (e. g. xanthan gum) and water ad 100 wt% to give a fine active substance suspension. Dilution with water gives a stable suspension of the active substance. For FS type composition up to 40 wt% binder (e. g. polyvinyl alcohol) is added.

### vi) Water-dispersible granules and water-soluble granules (WG, SG)

50-80 wt% of a compound I are ground finely with addition of dispersants and wetting agents (e. g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.

### vii) Water-dispersible powders and water-soluble powders (WP, SP, WS)

50-80 wt% of a compound I are ground in a rotor-stator mill with addition of 1-5 wt% dispersants (e. g. sodium lignosulfonate), 1-3 wt% wetting agents (e. g. alcohol ethoxylate) and solid carrier (e. g. silica gel) ad 100 wt%. Dilution with water gives a stable dispersion or solution of the active substance.

### viii) Gel (GW, GF)

In an agitated ball mill, 5-25 wt% of a compound I are comminuted with addition of 3-10 wt% dispersants (e. g. sodium lignosulfonate), 1-5 wt% thickener (e. g. carboxymethyl cellulose) and water ad 100 wt% to give a fine suspension of the active substance. Dilution with water gives a stable suspension of the active substance.

### ix) Microemulsion (ME)

5-20 wt% of a compound I are added to 5-30 wt% organic solvent blend (e. g. fatty acid dimethyl amide and cyclohexanone), 10-25 wt% surfactant blend (e. g. alcohol ethoxylate and arylphenol ethoxylate), and water ad 100 %. This mixture is stirred for 1 h to produce spontaneously a thermodynamically stable microemulsion.

### x) Microcapsules (CS)

An oil phase comprising 5-50 wt% of a compound I, 0-40 wt% water insoluble organic solvent (e. g. aromatic hydrocarbon), 2-15 wt% acrylic monomers (e. g. methylmethacrylate, methacrylic acid and a di- or triacrylate) are dispersed into an aqueous solution of a protective colloid (e. g. polyvinyl alcohol). Radical polymerization results in the formation of poly(meth)acrylate microcapsules. Alternatively, an oil phase comprising 5-50 wt% of a compound I according to the invention, 0-40 wt% water insoluble organic solvent (e. g. aromatic hydrocarbon), and an isocyanate monomer (e. g. diphenylmethene-4,4'-diisocyanatae) are dispersed into an aqueous solution of a protective colloid (e. g. polyvinyl alcohol). The addition of a polyamine (e. g. hexamethylenediamine) results in the formation of polyurea microcapsules. The monomers amount to 1-10 wt%. The wt% relate to the total CS composition.

### xi) Dustable powders (DP, DS)

1-10 wt% of a compound I are ground finely and mixed intimately with solid carrier (e. g. finely divided kaolin) ad 100 wt%.

### xii) Granules (GR, FG)

0.5-30 wt% of a compound I is ground finely and associated with solid carrier (e. g. silicate) ad 100 wt%. Granulation is achieved by extrusion, spray-drying or fluidized bed.

### xiii) Ultra-low volume liquids (UL)

1-50 wt% of a compound I are dissolved in organic solvent (e. g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions comprising generally comprise between 0.01 and 95%, preferably between 0.1 and 90%, and in particular between 0.5 and 75%, by weight of the compounds of the formula (I). The compounds of the formula (I) are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The agrochemical compositions in question give, after two-to-tenfold dilution, active substance concentrations of from 0.01 to 60% by weight, preferably from 0.1 to 40% by weight, in the ready-to-use preparations. Application can be carried out before or during sowing.

Methods for applying compounds of the formula (I), agrochemical compositions thereof, on to plant propagation material, especially seeds, include dressing, coating, pelleting, dusting, soaking and in-furrow application methods of the propagation material. Preferably, compounds of the formula (I), agrochemical compositions thereof, respectively, are applied on to the plant propagation material by a method such that germination is not induced, e. g. by seed dressing, pelleting, coating and dusting.

Various types of oils, wetters, adjuvants, fertilizer, or micronutrients may be added to the compounds of the of formula (I), the agrochemical compositions comprising them as premix or, if appropriate not until immediately prior to use (tank mix). These agents can be admixed with the agrochemical compositions according to the invention in a weight ratio of 1:100 to 100:1, preferably 1:10 to 10:1.

The user applies the compounds of the of formula (I) according to the invention, the agrochemical compositions comprising them usually from a pre-dosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, either individual components of the agrochemical composition according to the invention or partially premixed components, e. g. components comprising compounds of the of formula (I), may be mixed by the user in a spray tank and further auxiliaries and additives may be added, if appropriate.

In a further embodiment, individual components of the agrochemical composition according to the invention such as parts of a kit or parts of a binary or ternary mixture may be mixed by the user himself in a spray tank and further auxiliaries may be added, if appropriate.

In a further embodiment, either individual components of the agrochemical composition according to the invention or partially premixed components, e. g components comprising compounds of the formula (I), can be applied jointly (e.g. after tank mix) or consecutively.

The compounds of the formula (I), are suitable as herbicides. They are suitable as such or as an appropriately formulated composition (agrochemical composition). The compounds of the formula (I), or the agrochemical compositions comprising the compounds of the formula (I), control vegetation on non-crop areas very efficiently, especially at high rates of application. They act against broad-leaved weeds and grass weeds in crops such as wheat, rice, maize, soya and cotton without causing any significant damage to the crop plants. This effect is mainly observed at low rates of application.

The compounds of the formula (I), or the agrochemical compositions comprising them, are applied to the plants mainly by spraying the leaves. Here, the application can be carried out using, for example, water as carrier by customary spraying techniques using spray liquor amounts of from about 100 to 1000 l/ha (for example from 300 to 400 l/ha). The compounds of the formula (I), or the agrochemical compositions comprising them, may also be applied by the low-volume or the ultra-low-volume method, or in the form of microgranules.

Application of the compounds of the formula (I), or the agrochemical compositions comprising them, can be done before, during and/or after, preferably during and/or after, the emergence of the undesirable plants.

The compounds of the formula (I), or the agrochemical compositions comprising them, can be applied pre-, post-emergence or pre-plant, or together with the seed of a crop plant. It is also possible to apply the compounds of the formula (I), or the agrochemical compositions comprising them, by applying seed, pretreated with the compounds of the formula (I), or the agrochemical compositions comprising them, of a crop plant. If the active ingredients are less well tolerated by certain crop plants, application techniques may be used in which the compositions are sprayed, with the aid of the spraying equipment, in such a way that as far as possible they do not come into contact with the leaves of the sensitive crop plants, while the active ingredients reach the leaves of undesirable plants growing underneath, or the bare soil surface (post-directed, lay-by).

In a further embodiment, the compounds of the formula (I), or the agrochemical compositions comprising them, can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the compounds of the formula (I), or the agrochemical compositions prepared therefrom. Here, the compositions can be applied diluted or undiluted.

The term "seed" comprises seed of all types, such as, for example, corns, seeds, fruits, tubers, seedlings and similar forms. Here, preferably, the term seed describes corns and seeds. The seed used can be seed of the useful plants mentioned above, but also the seed of transgenic plants or plants obtained by customary breeding methods.

When employed in plant protection, the amounts of active substances applied, i.e. the compounds of the formula (I), without formulation auxiliaries, are, depending on the kind of effect desired, from 0.001 to 2 kg per ha, preferably from 0.005 to 2 kg per ha, more preferably from 0.025 to 1 kg per ha and in particular from 0.05 to 0.75 kg per ha.

In another embodiment of the invention, the application rate of the compounds of the formula (I) is from 0.001 to 3 kg/ha, preferably from 0.005 to 2.5 kg/ha and in particular from 0.01 to 2 kg/ha of active substance (a.s.).

In another preferred embodiment of the invention, the rates of application of the compounds of the formula (I) according to the present invention (total amount of compounds of the formula (I) are from 0.1 g/ha to 3000 g/ha, preferably 10 g/ha to 1000 g/ha, depending on the control target, the season, the target plants and the growth stage.

In another preferred embodiment of the invention, the application rates of the compounds of the formula (I) are in the range from 0.1 g/ha to 5000 g/ha and preferably in the range from 1 g/ha to 2500 g/ha or from 5 g/ha to 2000 g/ha.

In another preferred embodiment of the invention, the application rate of the compounds of the formula (I) is 0.1 to 1000 g/ha, preferably1 to 750 g/ha, more preferably 5 to 500 g/ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

In another embodiment of the invention, to treat the seed, the amounts of active substances applied, i.e. the compounds of the formula (I) are generally employed in amounts of from 0.001 to 10 kg per 100 kg of seed.

In case of compositions according to the present invention it is immaterial whether the compounds of the formula (I) are formulated and applied jointly or separately.

In the case of separate application it is of minor importance, in which order the application takes place. It is only necessary, that the compounds of the formula (I) are applied in a time frame that allows simultaneous action of the active ingredients on the plants, preferably within a time-frame of at most 14 days, in particular at most 7 days.

Depending on the application method in question, the compounds of the formula (I), or the agrochemical compositions comprising them, can additionally be employed in a further number of crop plants for eliminating undesirable plants. Examples of suitable crops are the following:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera and Zea mays.

Preferred crops are Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera and Zea mays.

Especially preferred crops are crops of cereals, corn, soybeans, rice, oilseed rape, sunflower, cotton, potatoes, peanuts or permanent crops, in particular cereals, more particularly *Triticum aestivum* (winter), *Triticum aestivum* (spring), *Triticum durum, Triticum spelta, Hordeum vulgare* (winter), *Hordeum vulgare* (spring), *Secale cereale* (winter), *Secale cereale* (spring), *Triticosecale* (winter), *Triticosecale* (spring).

The compounds of the formula (I) according to the invention, or the agrochemical compositions comprising them, can also be used in genetically modified plants. The term "genetically modified plants" is to be understood as plants whose genetic material has been modified by the use of recombinant DNA techniques to include an inserted sequence of DNA that is not native to that plant species' genome or to exhibit a deletion of DNA that was native to that species' genome, wherein the modification(s) cannot readily be obtained by cross breeding, mutagenesis or natural recombination alone. Often, a particular genetically modified plant will be one that has obtained its genetic modification(s) by inheritance through a natural breeding or propagation process from an ancestral plant whose genome was the one directly treated by use of a recombinant DNA technique. Typically, one or more genes have been integrated into the genetic material of a genetically modified plant in order to improve certain properties of the plant. Such genetic modifications also include but are not limited to targeted post-translational modification of protein(s), oligo- or polypeptides. e. g., by inclusion therein of amino acid mutation(s) that permit, decrease, or promote glycosylation or polymer additions such as prenylation, acetylation farnesylation, or PEG moiety attachment.

Plants that have been modified by breeding, mutagenesis or genetic engineering, e.g. have been rendered tolerant to applications of specific classes of herbicides, such as auxin herbicides such as dicamba or 2,4-D; bleacher herbicides such as hydroxyphenylpyruvate dioxygenase (HPPD) inhibitors or phytoene desaturase (PDS) inhibitors; acetolactate synthase (ALS) inhibitors such as sulfonyl ureas or imidazolinones; enolpyruvyl shikimate 3-phosphate synthase (EPSP) inhibitors such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering; furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are, for example, described in Pest Management Science 61, 2005, 246; 61, 2005, 258; 61, 2005, 277; 61, 2005, 269; 61, 2005, 286; 64, 2008, 326; 64, 2008, 332; Weed Science 57, 2009, 108; Australian Journal of Agricultural Research 58, 2007, 708; Science 316, 2007, 1185; and references quoted therein. Several cultivated plants have been rendered tolerant to herbicides by mutagenesis and conventional methods of breeding, e. g., Clearfield® summer rape (Canola, BASF SE, Germany) being tolerant to imidazolinones, e. g., imazamox, or ExpressSun® sunflowers (DuPont, USA) being tolerant to sulfonyl ureas, e. g., tribenuron. Genetic engineering methods have been used to render cultivated plants such as soybean, cotton, corn, beets and rape, tolerant to herbicides such as glyphosate, imidazolinones and glufosinate, some of which are under development or commercially available under the brands or trade names RoundupReady® (glyphosate tolerant, Monsanto, USA), Cultivance® (imidazolinone tolerant, BASF SE, Germany) and LibertyLink® (glufosinate tolerant, Bayer CropScience, Germany).

Furthermore, plants are also covered that are by the use of recombinant DNA techniques capable to synthesize one or more insecticidal proteins, especially those known from the bacterial genus Bacillus, particularly from Bacillus thuringiensis, such as delta-endotoxins, e. g., CryIA(b), CryIA(c), CryIF, CryIF(a2), CryIIA(b), CryIIIA, CryIIIB(b1) or Cry9c; vegetative insecticidal proteins (VIP), e. g., VIP1, VIP2, VIP3 or VIP3A; insecticidal proteins of bacteria colonizing nematodes, e. g., Photorhabdus spp. or Xenorhabdus spp.; toxins produced by animals, such as scorpion toxins, arachnid toxins, wasp toxins, or other insect-specific neurotoxins; toxins produced by fungi, such as Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as including pre-toxins, hybrid proteins, truncated or otherwise modified proteins. Hybrid proteins are characterized by a new combination of protein domains, (see, e. g., WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g., in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes

(Nematoda). Genetically modified plants capable to synthesize one or more insecticidal proteins are, e. g., described in the publications mentioned above, and some of which are commercially available such as YieldGard® (corn cultivars producing the Cry1Ab toxin), YieldGard® Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme Phosphinothricin-N-Acetyltransferase [PAT]); NuCOTN® 33B (cotton cultivars producing the Cry1Ac toxin), Bollgard® I (cotton cultivars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, KnockOut®, BiteGard®, Protecta®, Bt11 (e. g., Agrisure® CB) and Bt176 from Syngenta Seeds SAS, France, (corn cultivars producing the Cry1Ab toxin and PAT enzyme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e.g., EP-A 392 225), plant disease resistance genes (e. g., potato culti-vars, which express resistance genes acting against Phytophthora infestans derived from the Mexican wild potato, Solanum bulbocastanum) or T4-lyso-zym (e.g., potato cultivars capable of synthesizing these proteins with increased resistance against bacteria such as Erwinia amylovora). The methods for producing such genetically modi-fied plants are generally known to the person skilled in the art and are described, e.g., in the publications mentioned above.

Furthermore, plants are also covered that are by the use of recombinant DNA techniques capable to synthesize one or more proteins to increase the productivity (e.g., bio-mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of ingredients or new ingredients, specifically to improve human or animal nutrition, e. g., oil crops that produce health-promoting long-chain omega-3 fatty acids or unsaturated omega-9 fatty acids (e. g., Nexera® rape, Dow AgroSciences, Canada). Furthermore, plants are also covered that contain by the use of recombinant DNA techniques a modified amount of ingredients or new ingredients, specifically to improve raw material production, e.g., potatoes that produce increased amounts of amylopectin (e.g. Amflora® potato, BASF SE, Germany).

Furthermore, it has been found that the compounds of the formula (I) according to the invention, or the agrochemical compositions comprising them, are also suitable for the defoliation and/or desiccation of plant parts, for which crop plants such as cotton, potato, oilseed rape, sunflower, soybean or field beans, in particular cotton, are suitable. In this regard, agrochemical compositions for the desiccation and/or defoliation of plants, processes for preparing these agrochemical compositions and methods for desiccating and/or defoliating plants using the compounds of the formula (I) have been found.

As desiccants, the compounds of the formula (I) are particularly suitable for desiccating the above-ground parts of crop plants such as potato, oilseed rape, sunflower and soybean, but also cereals. This makes possible the fully mechanical harvesting of these important crop plants.

Also of economic interest is to facilitate harvesting, which is made possible by concentrating within a certain period of time the dehiscence, or reduction of adhesion to the tree, in citrus fruit, olives and other species and varieties of pernicious fruit, stone fruit and nuts. The same mechanism, i.e. the promotion of the development of abscission tissue between fruit part or leaf part and shoot part of the plants is also essential for the controlled defoliation of useful plants, in particular cotton.

Moreover, a shortening of the time interval in which the individual cotton plants mature leads to an increased fiber quality after harvesting.

### I. Synthesis example

The compounds of the formula (I) can be prepared according to the methods outlined below and according to procedures that are set forth in US 435077, US 435076, JP 8193011 and JP 8291146. 3-amino-phenol (CAS-Nr. 591-27-5) and 2-chloro-5-fluoropyrimidine (CAS-Nr. 62802-42-0) are commercially available.

### I.1 Preparation of 3-(5-fluoropyrimidin-2-yl)oxy-aniline

To a solution of 3-aminophenol (25.0 g) in dimethylsulfoxide (300 mL) was added potassium carbonate (63.3 g) and 2-chloro-5-fluoropyrimidine (30.4 g). The reaction was stirred at 80°C for 4 hours, quenched with water and extracted with ethyl acetate. The combined organic phases were washed with brine, dried (magnesium sulfate), filtered and concentrated. Purification of this material was accomplished by flash column chromatography eluting with 30 % ethyl acetate/cyclohexane. The product containing fractions were collected and concentrated to give the title compound.

### I.2 Preparation of 1,1,1-trifluoro-N-[3-(5-fluoropyrimidin-2-yl)oxy-phenyl]methanesulfonamide (compound E-1)

To a solution of 3-(5-fluoropyrimidin-2-yl)oxy-aniline (23.3 g) in dichloromethane (200 mL) was added at a temperature of 0 °C to 5 °C Triethylamine (31.5 mL) followed by drop wise addition of trifluoromethanesulfonic acid anhydride (38.1 mL). The reaction was stirred for 1 hour. A solution of sodium hydroxide in water (2 molar, 170 mL) and methanol (100 mL) was added and the reaction mixture was stirred for 2 hours at room temperature. The reaction was quenched with hydrochloric acid, diluted with water and extracted with dichloromethane. The combined organic phases were dried (magnesium sulfate), filtered and concentrated. Purification of this material was accomplished by flash column chromatography eluting with 15% ethyl acetate/cyclohexane. The product containing fractions were collected and concentrated to give the title compound.

### I.3 Preparation of N-[3-(5-fluoropyrimidin-2-yl)oxy-phenyl]-N-(trifluoromethylsulfonyl)acetamide (compound E-21)

To a solution of 1,1,1-trifluoro-N-[3-(5-fluoropyrimidin-2-yl)oxy-phenyl]methanesulfonamide (500 mg) in tetrahydrofuran (5.0 mL) was added at a temperature of 0 °C to 5 °C sodium hydride (70.0 mg) and the reaction mixture was stirred for 10 minutes. A solution of acetylchloride (0.120 ml) in tetrahydrofuran (5.0 mL) was added slowly and the reaction was stirred at room temperature over night. Subsequently the reaction was quenched with water and extracted with ethyl acetate. The combined organic phases were dried (magnesium sulfate), filtered and concentrated. Purification of this material was accomplished by flash column chromatography eluting with 10% ethyl acetate/cyclohexane. The product containing fractions were collected and concentrated to give the title compound.

The other compounds listed in Table E were prepared in an analogous manner.

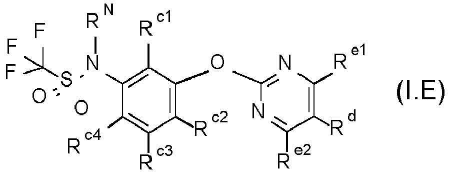

**Table E: Compounds of the formula (I.E)**

| **Ex. no** | **R^{N}** | **R^{c1}** | **R^{c2}** | **R^{c3}** | **R^{c4}** | **R^{e1}** | **R^{e2}** | **R^{d}** | **HPLC Rt (min)** |
|---|---|---|---|---|---|---|---|---|---|
| E-1 | H | H | H | H | H | H | H | F | 1.035* |
| E-2 | H | H | H | H | H | H | H | Cl | 1.094* |
| E-3 | H | H | H | H | H | H | H | Br | 1.092* |
| E-4 | H | Me | H | H | H | H | H | F | 1.077* |
| E-5 | H | H | Me | H | H | H | H | F | 1.072* |
| E-6 | H | H | H | Me | H | H | H | F | 1.099* |
| E-7 | H | H | H | H | Me | H | H | F | 1.090* |
| E-8 | H | Me | Me | H | H | H | H | F | 1.155* |
| E-9 | H | Me | H | H | Me | H | H | F | 1.121* |
| E-10 | H | H | Me | H | Me | H | H | F | 1.108* |
| E-11 | H | MeO(C=O) | H | H | H | H | H | F | 1.026* |
| E-12 | H | H | MeO(C=O) | H | H | H | H | F | 1.181* |
| E-13 | H | H | H | MeO(C=O) | H | H | H | F | 1.077* |
| E-14 | H | H | H | H | MeO(C=O) | H | H | F | 1.041 * |
| E-15 | H | H | F | H | H | H | H | F | 1.019* |
| E-16 | H | H | H | H | F | H | H | F | 1.066* |
| E-17 | H | F | H | H | H | H | H | F | 1.051 * |
| E-18 | H | Cl | H | H | H | H | H | F | 1.054* |
| E-19 | H | H | H | H | H | Me₂N | H | F | 1.133** |
| E-20 | H | H | H | H | H | MeS | H | F | 1.168* |
| E-21 | Me(C=O) | H | H | H | H | H | H | F | 1.084* |

| | | | | | | | | | |
|---|---|---|---|---|---|---|---|---|---|
| * HPLC: HPLC-column Kinetex XB C18 1,7µ (50 x 2,1 mm); eluent: acetonitrile / water + 0.1 % trifluoroacetic acid (gradient from 5:95 to 100 : 0 in 1.5 min at 60°C, flow gradient from 0.8 to 1.0 ml/min in 1.5 min). MS: Quadrupol Electrospray lonisation, 80 V (positive mode). Rₜ: retention time in minutes. ** HPLC: HPLC-column Kinetex XB C18 1,7µ (50 x 2,1 mm); eluent: acetonitrile / water + 0.1 % formic acid (gradient from 5:95 to 100 : 0 in 1.5 min at 60°C, flow gradient from 0.8 to 1.0 ml/min in 1.5 min). MS: Quadrupol Electrospray lonisation, 80 V (negative mode). Rₜ: retention time in minutes. | | | | | | | | | |

### II. Use examples

The herbicidal activity of the fluoromethanesulfonamides of the formula (I) was demonstrated by the following greenhouse experiments:
The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the plants had rooted. This cover caused uniform germination of the test plants, unless this had been impaired by the active ingredients.

For the post-emergence treatment, the test plants were first grown to a height of 3 to 15 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment. Depending on the species, the plants were kept at 10 - 25°C or 20 - 35°C, respectively. The test period extended over 2 to 4 weeks. During this time, the plants were tended, and their response to the individual treatments was evaluated.

Evaluation was carried out using a scale from 0 to 100. 100 means no emergence of the plants, or complete destruction of at least the aerial moieties, and 0 means no damage, or normal course of growth. A very good herbicidal activity is given at values of at least 80.

The plants used in the greenhouse experiments were of the following species:

| **Bayer code** | **Scientific name** |
|---|---|
| ALOMY | Alopecurus myosuroides |
| APESV | Apera spica-venti |
| SETVI | Setaria viridis |
| ECHCG | Echinochloa crus-galli |

At an application rate of 500 g/ha, E-1, E-2, E-3, E-4, E-11, E-15, E-16, E-17, E-21, applied by the **post-emergence** method, showed herbicidal activity of at least 80 on the above mentioned scale against Alopecurus myosuroides.

At an application rate of 500 g/ha, E-1, E-2, E-4, E-5, E-6, E-7, E-9, E-10, E-11, E-15, E-18, E-21, applied by the **post-emergence** method, showed herbicidal activity of at least 80 on the above mentioned scale against Echinochloa crus-galli.

At an application rate of 500 g/ha, E-1, E-2, E-3, E-4, E-6, E-7, E-9, E-11, E-13, E-15, E-17, E-18, E-21, applied by the **post-emergence** method, showed herbicidal activity of at least 80 on the above mentioned scale against Setaria viridis.

At an application rate of 500 g/ha, E-1, E-3, E-4, E-6, E-7, E-9, E-10, E-15, E-18, E-21, applied by the **pre-emergence** method, showed herbicidal activity of at least 80 on the above mentioned scale against Apera spica-venti.

## Claims

1. Compounds of the formula (I) or their N-oxides or their salts or acid addition salts, 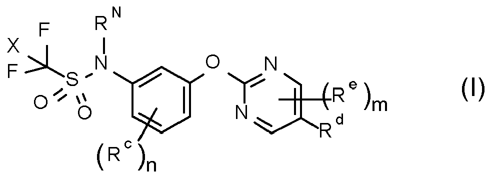 wherein:
X is hydrogen, halogen or C₁-C₆-haloalkyl;
R^{N} is hydrogen, C₁-C₆-alkyl, C₁-C₆-alkoxy-C₁-C₆-alkyl, C₂-C₆-alkenyl, C₂-C₆-alkynyl, C₃-C₆-cycloalkyl, phenyl-C₁-C₄-alkyl, heteroaryl-C₁-C₄-alkyl, (C=O)-(C₁-C₆-alkyl), (C=O)- C₂-C₆-alkynyl, (C=O)-(C₃-C₆-cycloalkyl), (C=O)-(C₁-C₆-alkoxy), (C=O)-(C₂-C₆-alkynyloxy), (C=O-(C₁-C₆-alkylthio), (C=O)-(C₁-C₆-alkylamino), (C=O)-(C₁-C₆-dialkylamino), (C=O)-phenyl, (C=O)-heteroaryl, (C=O)-(phenyl-C₁-C₄-alkyl), (C=O)-(heteroaryl-C₁-C₄-alkyl), C₁-C₆-alkylsulfonyl, C₃-C₆-cycloalkylsulfonyl, C₁-C₆-alkoxysulfonyl, C₁-C₆-alkylaminosulfonyl, C₁-C₆-dialkylaminosulfonyl, phenylsulfonyl, heteroarylsulfonyl, C₁-C₆-alkyl-(C=O)-O-C₁-C₆-alkyl, C₃-C₆-cycloalkyl-(C=O)-O-C₁-C₆-alkyl, C₁-C₆-haloalkyl-(C=O)-O-C₁-C₆-alkyl, C₂-C₆-alkenyl-(C=O)-O-C₁-C₆-alkyl, C₂-C₆-alkynyl-(C=O)-O-C₁-C₆-alkyl, C₁-C₆-alkoxy-(C=O)-O-C₁-C₆-alkyl, C₁-C₆-haloalkoxy-(C=O)-O-C₁-C₆-alkyl, C₂-C₆-alkenyloxy-(C=O)-O-C₁-C₆-alkyl or C₂-C₆-alkynyloxy-(C=O)-O-C₁-C₆-alkyl; and wherein the aliphatic and cyclic moieties of R^{N} are unsubstituted or substituted by 1, 2, 3 or up to the maximum number of identical or different groups R^{N1}; wherein
R^{N1} is halogen, hydroxy, cyano, nitro, C₁-C₄-alkyl, C₁-C₄-alkoxy, C₁-C₄-alkylthio, C₁-C₄-alkylsulfinyl, C₁-C₄-alkylsulfonyl, C₁-C₄-alkylamino, C₁-C₄-dialkylamino, C₃-C₆-cycloalkyl, C₁-C₄-haloalkyl or C₁-C₄-haloalkoxy;
R^{c} is independently selected from the group consisting of halogen, cyano, nitro, hydroxy, SH, NH₂, C₁-C₆-alkyl, C₁-C₆-alkoxy, C₁-C₆-haloalkyl, C₁-C₆-haloalkoxy, C₁-C₄-alkoxy-C₁-C₄-alkyl, C₁-C₆-alkylthio, C₁-C₆-haloalkylthio, C₁-C₆-alkylsulfinyl, C₁-C₆-alkylsulfonyl, C₁-C₆-haloalkylsulfonyl, C₁-C₆-alkylamino, C₁-C₆-dialkylamino, C₁-C₆-alkyl-(C=O)-amino, C₁-C₆-alkylsulfonylamino, C₁-C₆-alkyl-(C=O)-(C₁-C₆-alkylamino), (C₁-C₆-alkylsulfonyl)-(C₁-C₆-alkylamino), C₂-C₆-alkenyl, C₂-C₆-alkynyl, C₂-C₆-alkenyloxy, C₂-C₆-alkynyloxy, C₃-C₈-cycloalkyl, C₃-C₈-cycloalkoxy, (C=O)-OH, (C=O)-NH₂, (C=O)-(C₁-C₆-alkyl), (C=O)-(C₁-C₆-alkenyl), (C=O)-(C₁-C₆-alkynyl), (C=O)-(C₁-C₆-alkoxy), (C=O)-(C₁-C₆-alkenyloxy), (C=O)-(C₁-C₆-alkynyloxy), (C=O-(C₁-C₆-alkylthio), (C=O)-(C₁-C₆-alkylamino), (C=O)-(C₁-C₆-dialkylamino), (C=N-O-(C₁-C₆-alkyl))-(C₁-C₆-alkyl), (CH=N-O-(C₁-C₆-alkyl)) and (C=N-O-(C₁-C₆-alkyl))-(C₁-C₆-alkoxy); and wherein the aliphatic and alicyclic moieties are unsubstituted or substituted by 1, 2, 3 or up to the maximum number of identical or different groups R^{c1}; wherein
R^{c1} is halogen, hydroxy, cyano, nitro, C₁-C₄-alkyl, C₁-C₄-alkoxy, C₁-C₄-alkylthio, C₁-C₄-alkylsulfinyl, C₁-C₄-alkylsulfonyl, C₁-C₄-alkylamino, C₁-C₄-dialkylamino, C₃-C₆-cycloalkyl, C₁-C₄-haloalkyl or C₁-C₄-haloalkoxy;
n is 0, 1, 2 or 3;
Re is independently selected from the group consisting of halogen, cyano, nitro, hydroxy, SH, NH₂, C₁-C₆-alkyl, C₁-C₆-alkoxy, C₁-C₆-haloalkyl, C₁-C₆-haloalkoxy, C1-C₄-alkoxy-C₁-C₄-alkyl, C₁-C₆-alkylthio, C₁-C₆-haloalkylthio, C₁-C₄-alkylthio-C₁-C₄-alkyl, C₁-C₆-alkylsulfinyl, C₁-C₆-alkylsulfonyl, C₁-C₆-alkylamino, C₁-C₆-dialkylamino, C₁-C₆-alkyl-(C=O)-amino, C₂-C₆-alkenyl, C₂-C₆-alkynyl, C₃-C₈-cycloalkyl, C₃-C₈-cycloalkoxy, (C=O)-OH, (C=O)-NH₂, (C=O)-(C₁-C₆-alkyl), (C=O)-(C₁-C₆-alkoxy), (C=O-(C₁-C₆-alkylthio), (C=O)-(C₁-C₆-alkylamino), (C=O)-(C₁-C₆-dialkylamino);
m is 0, 1 or 2;
R^{d} is independently selected from the group consisting of fluorine, chlorine or bromine.

2. Compounds according to claim 1, wherein X is hydrogen, chlorine or fluorine.

3. Compounds according to claim 1 or 2, wherein X is fluorine.

4. Compounds according to any one of claims 1 to 3, wherein R^{N} is hydrogen.

5. Compounds according to any one of claims 1 to 4, wherein n and m are 0.

6. An agrochemical composition, which comprises at least one auxiliary and at least one compound of the formula (I), or an N-oxide, or a salt thereof, or an acid addition salt thereof, according to any one of claims 1 to 5.

7. An agrochemical composition according to claim 6 further comprising seed, wherein the amount of the compound of the formula (I), or an N-oxide, or a salt thereof or an acid addition salt thereof, is from 0.1 g to 10 kg per 100 kg of seed.

8. The use of compounds of the formula (I) according to any one of claims 1 to 5, their N-oxides or their salts or their acid addition salts, for controlling unwanted vegetation.

9. A method for controlling unwanted vegetation, which comprises allowing a herbicidally effective amount of at least one compound of the formula (I), or an N-oxide, or a salt thereof, or an acid addition salt thereof, according to any one of claims 1 to 5, or a composition according to claim 6 to act on plants, their seeds and/or their habitat.
